# EUROPEAN PATENT SPECIFICATION

(11) **EP 0 463 059 B1**
(45) Date of publication and mention of the grant of the patent: **30.12.1998**
(21) Application number: 90905020.5
(22) Date of filing: 09.03.1990
(51) Int. Cl.: C07K 14/415, C07K 16/16, G01N 33/68, A61K 39/36

(54) **ALLERGENIC PROTEINS FROM RAGWEED AND USES THEREFOR**
ALLERGENE PROTEINE AUS AMBROSIA UND VERWENDUNG DAVON
PROTEINES ALLERGENES TIREES DE L'AMBROSIE ET UTILISATIONS DE CES PROTEINES

(30) Priority: 17.03.1989 US 325365
(43) Date of publication of application: 02.01.1992
(60) Divisional application: 96202477.4
(73) Proprietor: IMMULOGIC PHARMACEUTICAL CORPORATION, Cambridge Massachusetts 02139 (US); THE UNIVERSITY OF NORTH CAROLINA AT CHAPEL HILL, Chapel Hill, NC 27599-4100 (US)
(72) Inventor: ROGERS, Bruce, Cambridge, MA 02138 (US); KLAPPER, David, G., Chapel Hill, NC 27514 (US); RAFNAR, Thorunn, Chapel Hill, NC 27514 (US); KUO, Mei-Chang, Winchester, MA 01890 (US)
(74) Representative: Holdcroft, James Gerald, Dr.
(86) International application number: US9001310
(87) International publication number: WO9011293

(56) References cited:
- Molecular Immunology, Volume 25, No. 4, 1988, Pergamon Press Plc, (Oxford, GB) J.J. SMITH et al.: "Monoclonal Antibodies to Denatured Ragweed Pollen Allergen Amb a I: Characterization, Specificity for the Denatured Allergen, and Utilization for the Isolation of Immunogenic Peptides of Amb a I", pages 355- 365
- Molecular Immunology, Volume 22, No. 8, 1985, Pergamon Press Ltd, (Oxford, GB), L. GOODFRIEND et al.: "Ra5G. A Homologue of Ra5 in Giant Ragweed Pollen: Isolation, HLA-DR-Associated Activity and Amino Acid Sequence", pages 899-906
- Immunogenetics, Volume 26, 1987, Springer-Verlag, (New York, US), D.G. MARSH et al.: "Immune Responsiveness to Ambrosia Artemisilifolia (Short Ragweed) Pollen Allergen Amb a VI (Ra6) is Associated with HLA-DR5 in Allergic Humans", pages 230-236 see page 230 and page 232, column 2

## Description

### Background of the Invention

Genetically predisposed individuals, who make up about 10% of the population, become hypersensitized (allergic) to antigens from a variety of environmental sources to which they are exposed. Those antigens that can induce immediate and/or delayed types of hypersensitivity in people are called allergens. King, T. P., Adv, Immun., 23:77-105 (1976). Anaphylaxis or atopy, which includes the symptoms of hay fever, asthma and hives, is one form of immediate allergy. It can be caused by a variety of atopic allergens, such as products of grasses, trees, weeds, animal dander, insects, and food, drugs and chemicals.

The antibodies involved in atopic allergy belong primarily to the IgE class of immunoglobulins. IgE binds to mast cells and basophils. Upon combination of a specific allergen with IgE bound to mast cells, the IgE is cross-linked on the cell surface, resulting in the physiological effects of IgE-antigen interaction. Degranulation results in release of, among other substances, histamine, heparin, a chemotactic factor for eosinophilic leukocytes and the leukotrienes, C4, D4 and E4, which cause prolonged constriction of bronchial smooth muscle cells. Hood, L.E. et al., Immunology, (2nd ed.), pp460-462, The Benjamin/Cumming Publishing Co., Inc. (1984). These released substances are the mediators which result in allergic symptoms caused by combination of IgE with a specific allergen. Through them, the effects of an allergen are manifested. Such effects may be systemic or local in nature, depending on the route by which the antigen entered the body and the pattern of deposition of IgE and mast cells. Local manifestations generally occur on epithelial surfaces at the location at which the allergen entered the body. Systemic effects can include anaphylaxis (anaphylactic shock), which is the result of an IgE-basophil response to circulating (intravascular) antigen.

One allergen of particular concern for many people is Antigen E or Amb a I, a poorly-defined constituent (or group of constituents) which is the major allergenic component(s) of short ragweed (Ambrosia artemisiifolia I. or Ambrosia elatior) pollen and the major cause of late summer hayfever in North America and Canada. Smith, J. J., et al., Mol. Immun, 25:355-364 (1988); King, T. P.; et al., Biochem., 3:458-468 (1964); King, T.P., Adv. Immun., 23:77-105 (1976). It has been estimated that, on average, as much as 13% of the total serum IgE in ragweed-sensitive individuals is specific for Amb a I. Zeiss, C. R., et al., J. Immun., 110:414-421 (1973).
Amb a I has been claimed to be an acidic, 38,000 molecular weight, non-glycosylated protein which is cleaved during extraction and chromatographic isolation into two non-covalently associated chains: an alpha chain of 26,000 molecular weight and a beta chain of 12,000 molecular weight. Knox, R. B., et al., Nature, 255:1066-1068 (1970); Knox, R. B., and Heslop-Harrison, J., J. Cell Sci., 6:1-27 (1970); King, T. P., Adv. Immun., 23:77-105 (1976); King, T. P., et al., Archs Biochem. Biophys., 212:127-135 (1981). The two-chain and the single chain forms of Amb a I, which are both highly reactive with IgE, are allergenically and antigenically related. King, T. P., et al., Biochemistry, 3:458-468 (1964). It has been shown, however, that several physical and chemical modifications of Amb a I cause a marked loss of antigen and allergenic activity. King, T. P., et al., Archs Biochem. Bipophys. , 212:127-135 (1981); King, T. P., et al., Immunochemistry, 11: 83-92 (1974).

Because ragweed pollen is the chief causative agent of late-summer hay fever in the eastern United States and Canada, it has been the subject of more studies by different laboratories than any other pollen allergen. King, T. P., Adv. Immun., 23:77-105 (1976). Despite extensive study, the immunochemical definition of Amb a I is still far from complete. Smith and co-workers have begun characterization of the epitope structure of Amb a I, using a series of murine monoclonal antibodies raised against purified, native Amb a I. Three non-overlapping, non-repeating antigenic sites were defined (sites A, B, and C) and monoclonal antibodies directed to sites A and B together resulted in inhibition of 80% of human IgE binding to Amb a I. The reactivity of the monoclonal antibodies used was greatly diminished when Amb a I was physically or chemically modified. Olsen, Ph.D. thesis, University of North Carolina, Chapel Hill (1986); Olson, J. R., and Klapper, D. C., J. Immun., 136: 2109-2115 (1986). They indicated that the two sites (A and B) are conformationally dependent epitopes. That is, they are either single structures which lose their conformation during modification or composite structures made up of two or more discontinuous peptides which are proximal in the native allergen but separate once the allergen has been modified. Smith, J. J., et al., Mol. Immun., 25:355-365 (1988). This document describes the production of mAb's which bind denatured Amb a I and reports that the mAb's to denatured Amb A I do not affec the immunoactivity of native Amb a I.

Despite the considerable attention ragweed allergens have received, definition or characterization of the structure(s) or component(s) of the allergen responsible for its adverse effects on people is far from complete and current desentization therapy involves treatment with a complex, ill-defined extract of ragweed pollen.

### Summary of the Invention

The present invention relates to the subject matter of the claims.

It has now been shown that Antigen E or Amb a I is not a single protein but, rather, a family or families of proteins to which ragweed-sensitive individuals react. Ragweed allergen preparations are heterogeneous in nature and may include, in addition to what is currently referred to as Amb a I or Antigen E, other ragweed components which are allergenic (i.e., cause the typical adverse effects observed in a ragweed-sensitive individual upon exposure to ragweed pollen). These may include, for example, what is referred to in the literature as Antigen K and referred to herein as Amb a II.

### Brief Description of the Drawings

Figure 1 is a schematic representation of several routes by which assessment of proteins or peptides present in Amb a I and DNA encoding such proteins or peptides have been identified isolated and characterized.

Figure 1A is a schematic representation of screening of an Amb a I or Antigen E preparation, using monoclonal antibodies and oligoprobes.

Figure 1B is a schematic representation of screening of a ragweed flowerhead λgt10 library. It also illustrates the use of cross-hybridization and polymerase chain reaction (PCR) methods to obtain full-length cDNA clones encoding Amb a I and Amb a II.

Figure 2 is the nucleotide sequence of the DNA insert of UNC Clone 1 (referred to as Amb a IA), which was isolated from a λgt11 library by screening with monoclonal antibodies specific for components of an Amb a I preparation.

Figure 3 is the nucleotide sequence of the DNA insert of UNC Clone 6 (referred to as Amb a IB), which was isolated from a λgt11 library by screening with monoclonal antibodies specific for components of an Amb a I preparation.

Figure 4 is the nucleotide sequence of the DNA insert of UNC Clone 15 (referred to as Amb a IC), which was isolated from a λgt11 library by screening with monoclonal antibodies specific for components of an Amb a I preparation.

Figure 5 is the nucleotide sequence of the cDNA insert of IPC Clone 1, which was isolated from a λgt10 cDNA library using an oligonucleotide probe whose sequence was deduced from an amino acid sequence known to be present in the ragweed allergen preparation Amb a I. The location of the sequence from which the sequence of the oligonucleotide probe was deduced is underlined.

Figure 6 is the nucleotide sequence of the cDNA insert of IPC Clone 5, which was isolated from a λgt10 cDNA library using an oligonucleotide probe whose sequence was deduced from an amino acid sequence known to be present in the ragweed allergen preparation Amb a I. The location of the sequence from which the sequence of the oligonucleotide probe was deduced is underlined.

Figure 7 is the nucleotide sequence of the cDNA insert of IPC Clone 6, which was isolated from a λgt10 cDNA library using an oligonucleotide probe whose sequence was deduced from an amino acid sequence known to be present in the ragweed allergen preparation Amb a I. The location of the sequence from which the sequence of the oligonucleotide probe was deduced is underlined.

Figure 8 is a schematic representation of open reading frame analysis of the DNA insert of IPC Clone 1.

Figure 9 is a schematic representation of open reading frame analysis of the DNA insert of IPC Clone 5.

Figure 10 is a schematic representation of open reading frame analysis of the DNA insert of IPC Clone 6.

Figure 11 is the nucleotide sequence and deduced amino acid sequence of a full length Amb a IA clone (related to UNC clone 1).

Figure 12 is the nucleotide sequence and deduced amino acid sequence of a full length Amb a IB clone (related to UNC clone 6).

Figure 13 is the nucleotide sequence and deduced amino acid sequence of a full length Amb a IC clone (related to UNC clone 15).

Figure 14 is the nucleotide sequence and deduced amino acid sequence of a full length Amb a ID clone.

Figure 15 is the nucleotide sequence and deduced amino acid sequence of a full length Amb a II clone.

Figure 16 is the composite amino acid sequences of the Amb a I and Amb a II multigene family showing regions of similarity as well as regions of disagreements.

Figure 17 is a photograph of a Western blot of affinity purified Amb a I treated with rabbit anti-Amb a I polyclonal antibody, JBlE3-4 anti-Amb aI monoclonal antibody or ragweed allergic patient sera.

Figure 18 is a photograph of a two dimensional gel of an aqueous extract of short ragweed pollen, separated on the basis of size and charge and stained with T.P. King's antibody, which recognizes Amb a I (goat polyclonal anti-Amb a I).

Figure 19 is a photograph of a Western blot of several E. coli-expressed recombinant Amb a I cDNAs treated with goat anti-Amb a I antibody.

Figure 20 is a photograph of a Western blot of several E. coli expressed recombinant Amb a I cDNAs treated with human allergic sera strained with anti-human IgE.

Figure 21 is a graphic representation of T cell proliferation responses of ragweed allergic patient PBMC toward an aqueous extract of short ragweed pollen, affinity purified Amb a I (B7) chromatographically purified Amb a I and E. coli lysate containing expressed recombinant Amb a I proteins.

### Detailed Description of the Invention

The present invention is based on an investigation of ragweed pollen allergens, particularly the preparation known as Amb a I (or Antigen E) from short ragweed, using several inter-related approaches, each described below. The terms Amb a I and Antigen E are used interchangeably. Such a preparation, obtained from ragweed pollen, is likely to contain other ragweed allergens, such as Antigen K or Amb a II. The possibility that such a preparation does contain other such allergens has been assessed and results demonstrate that this is the case.

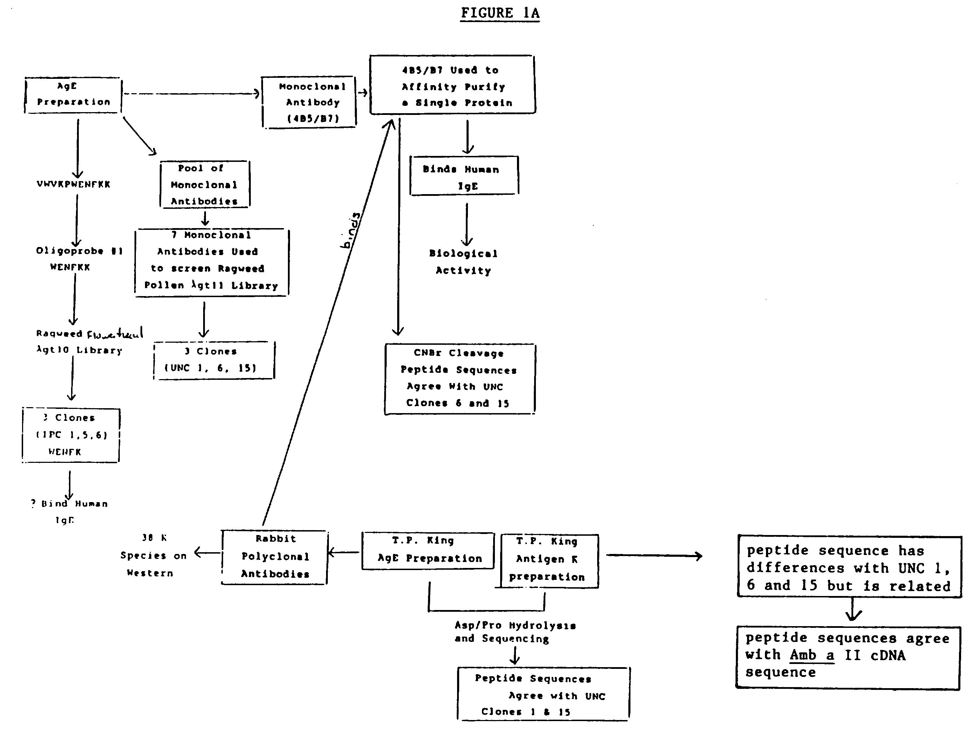

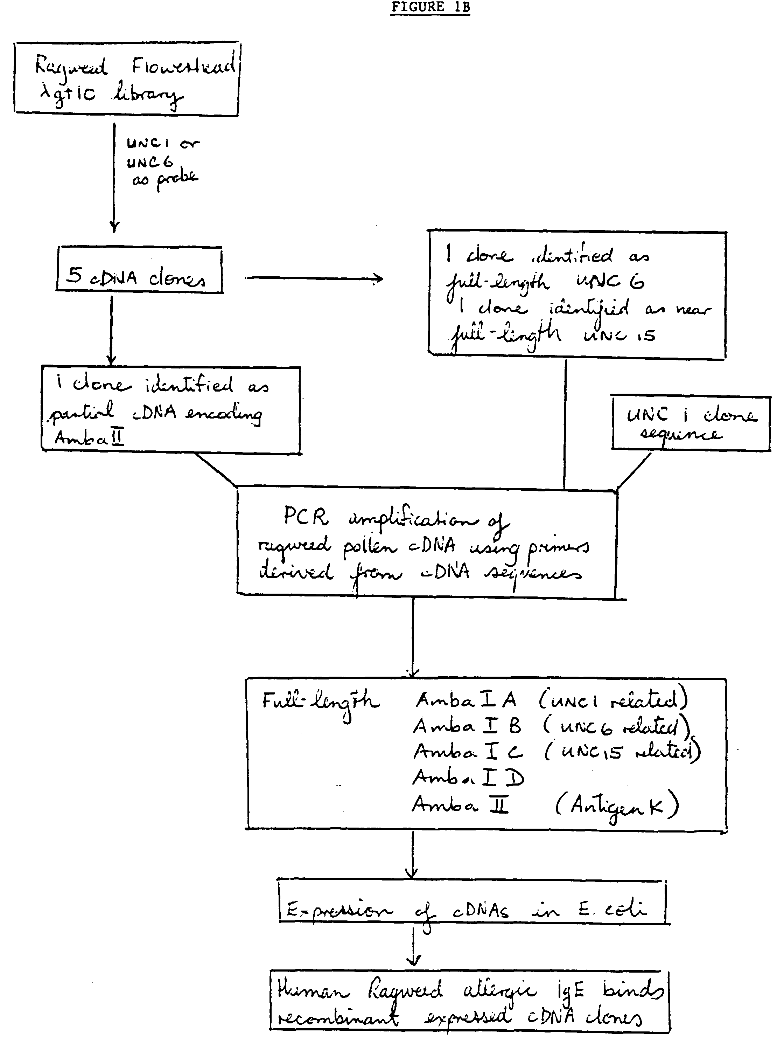

Results of work described herein show that Amb a I is not a single protein or peptide but is, in fact, heterogeneous in nature. That is, what is presently referred to as Antigen E (or Amb a I) appears to be a family or families of proteins or to be polymorphic in nature. The work described herein has resulted in identification and isolation of DNAs encoding peptides or amino acid sequences present in a ragweed allergen. As described, full-length cDNAs encoding Amb a IA, Amb a IB, Amb a IC and Amb a ID and Amb a II have been isolated and sequenced. It has also resulted in isolation and purification from an Amb a I preparation of a protein shown to bind human ragweed IgE and to bind rabbit Amb a I antisera produced using a purified Amb a I preparation. Interrelationships among DNAs and proteins or peptides identified and isolated using the approaches described in the following section have been demonstrated. For ease of presentation, the several approaches used are represented schematically in Figure 1A and 1B, to which reference is made in the following discussion.

As a result of the work described herein, DNAs encoding proteins or peptides present in ragweed allergens have been identified and isolated and the amino acid sequence of the encoded product has been deduced. In addition, through the use of monoclonal antibodies specific for Amb a I or Antigen E, a protein has been obtained from an Amb a I preparation. This protein, referred to as affinity purified Amb a I, has been shown to have biological activity (human IgE binding ability and ability to bind rabbit Amb a I antisera) and, thus, is highly likely to be an allergen. It has also been shown to be encoded by a region of the nucleotide sequences present in two of the isolated DNAs.

The following is a description of several approaches which have been used to identify and isolate DNAs encoding proteins or peptides from Amb a I or Antigen E preparations, as well as to isolate from an Amb a I preparation a protein shown to have Amb a I activity. As represented in Figure 1A an Amb a I or Antigen E preparation, which was prepared from pollen extract by a method based on the method of T.P. King and co-workers, was produced. King, T.P. et al, Arch. Bioch. and Biophys., 212:127-135 (1981). A panel of monoclonal antibodies produced by Klapper and co-workers was used to identify proteins in the preparation. Smith, J.J. et al., Mol. Immun., 25:355-365 (1988). Sequences of several peptides from an Antigen E preparation were determined by conventional techniques.

The following sections describe: 1) use of a pool of these monoclonal antibodies (i.e., a pool of monoclonal antibodies reactive with Amb a I) to identify clones containing DNA inserts encoding the reactive product and 2) use of an oligonucleotide probe, constructed from an amino acid sequence present in the Amb a I preparation to identify clones containing DNA inserts encoding the amino acid sequence. Each approach resulted in identification of three clones containing DNA encoding an amino acid sequence present in the Amb a I or Antigen E preparation. The two sets of clones isolated as described below have been shown to be different from each other.

### Use of Monoclonal Antibodies to Identify Clones Containing DNA Inserts Encoding Ragweed Protein

A pool of seven monoclonal antibodies specifically reactive with components of the Amb a I preparation was used to screen a ragweed pollen λgt11 library, using a known method. Young, R.A. and R.W. Davis, Proceedings of the National Academy of Sciences USA, 80: 1194.1198 (1983). This resulted in identification of three clones, initially designated UNC Clones 1, 6 and 15 and referred to herein as Amb a IA, IB and IC, respectively, which expressed a product recognized by at least one of the monoclonal antibodies in the panel. The nomenclature of cDNAs encoding the allergens Amb a I and Amb a II have been named according to the recommendations of the International Union of Immunological Societies Sub-Committee for Allergen Nomenclature (Marsh et al., Annals of Allergy, 60:699-506 (1988)).

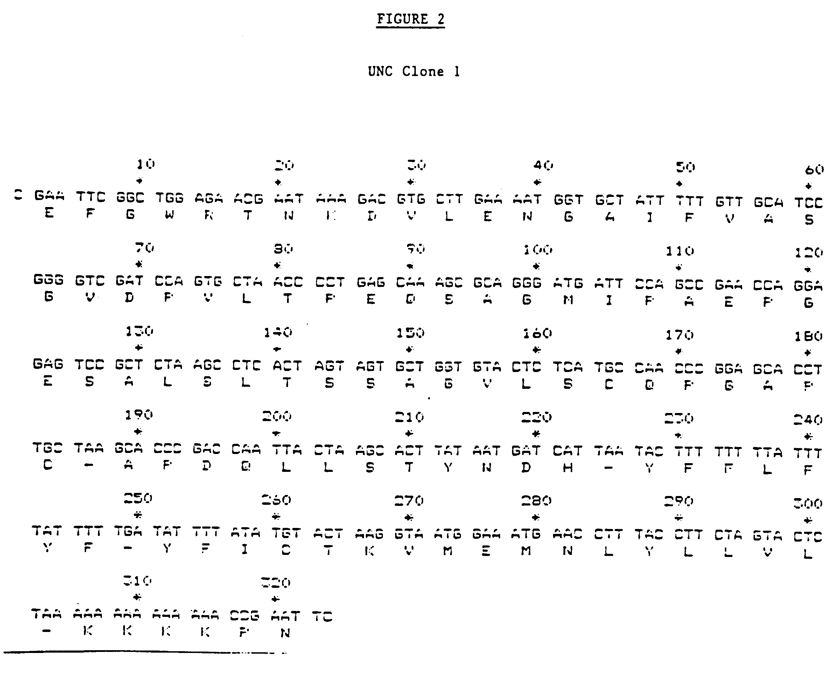

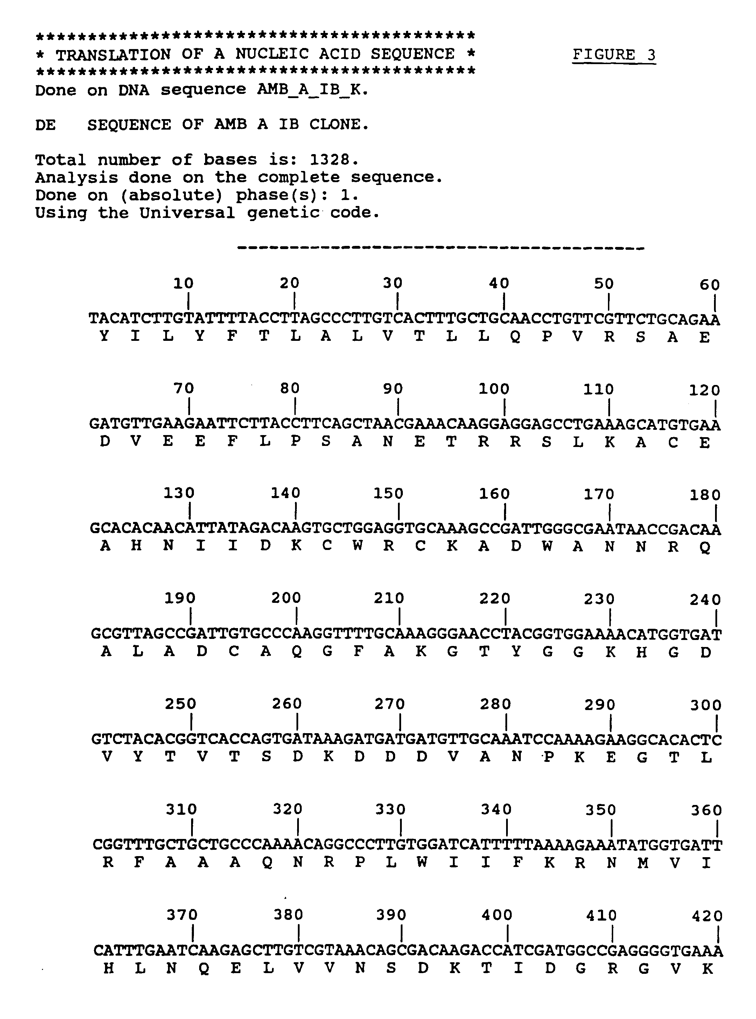

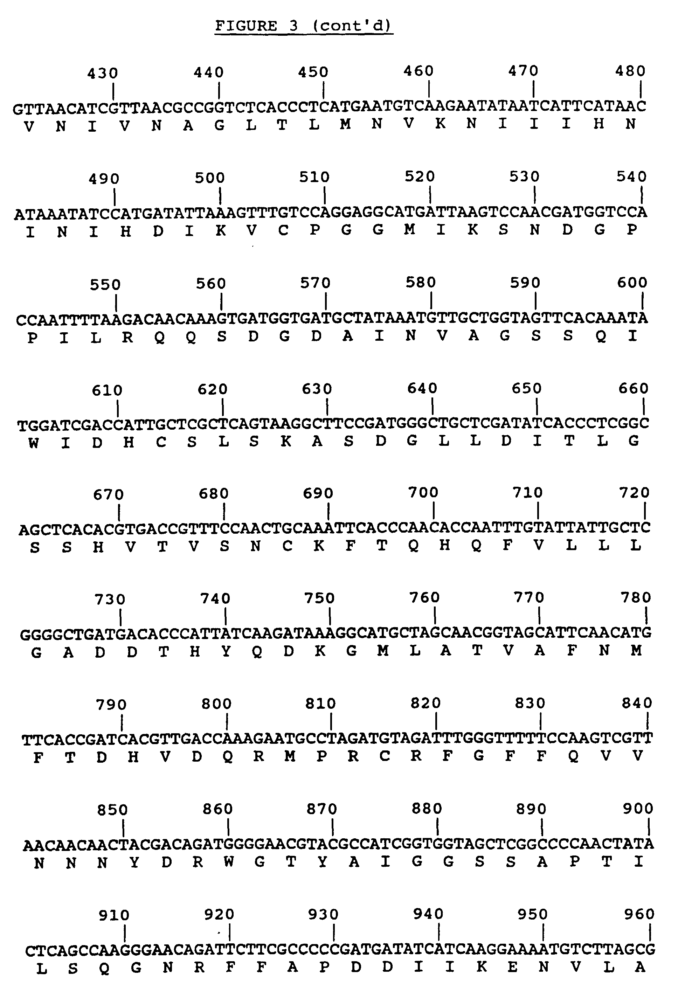

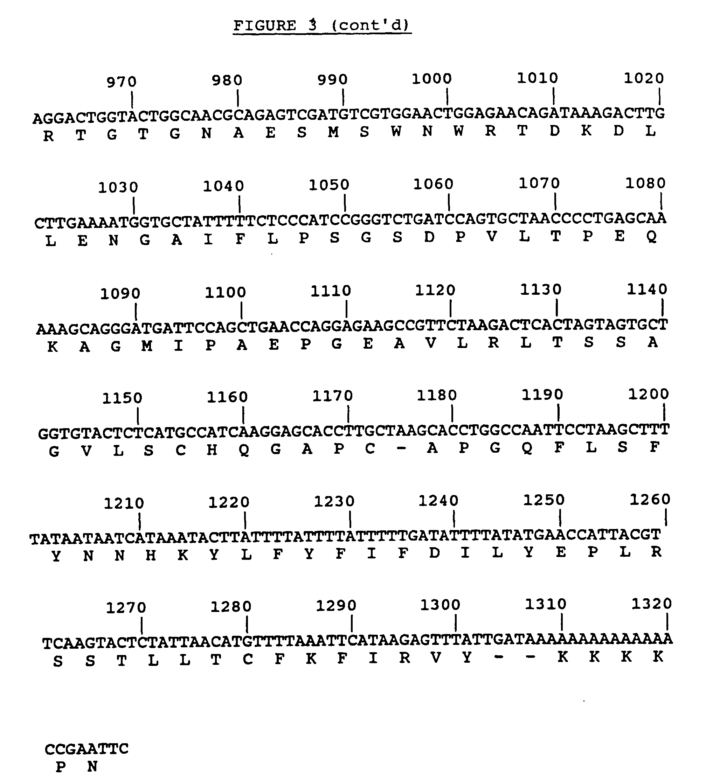

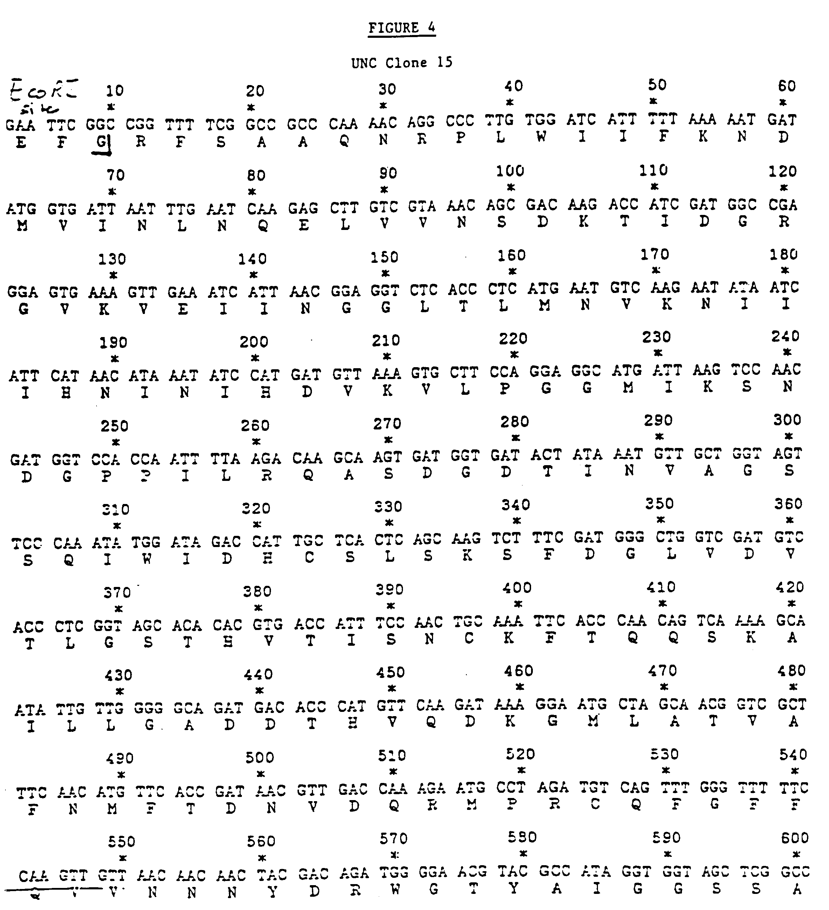

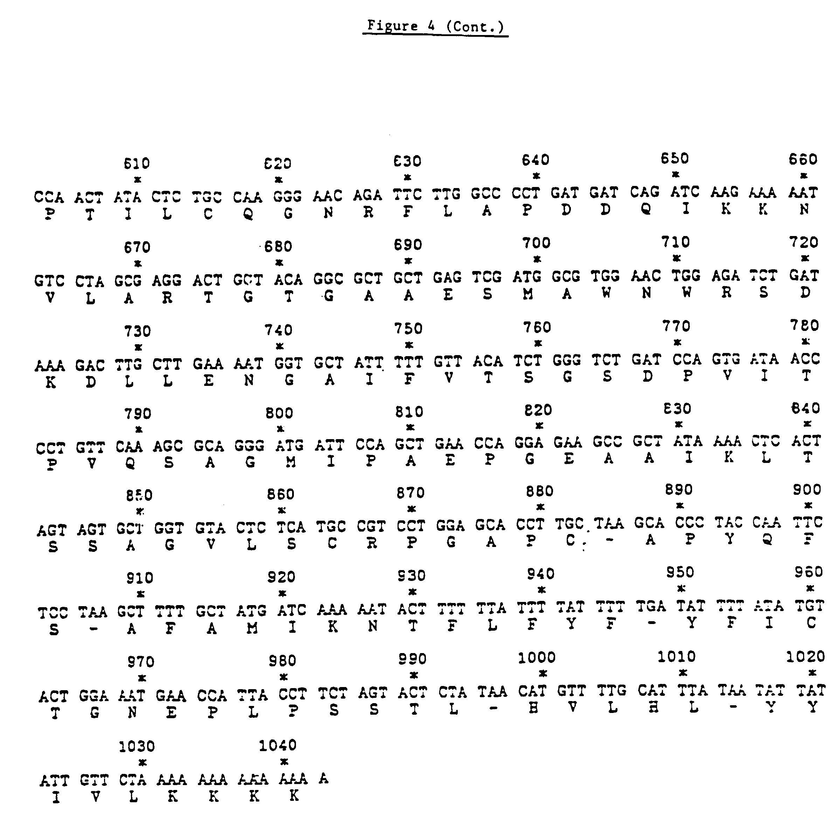

DNA isolated from the three reactive clones was sequenced, using the method of Sanger, F. et al. Sanger, F. et al., Proc. Natl. Acad. Sci., USA, 74:5463 (1977). The nucleotide sequences of the three clones are presented in Figures 2-4.

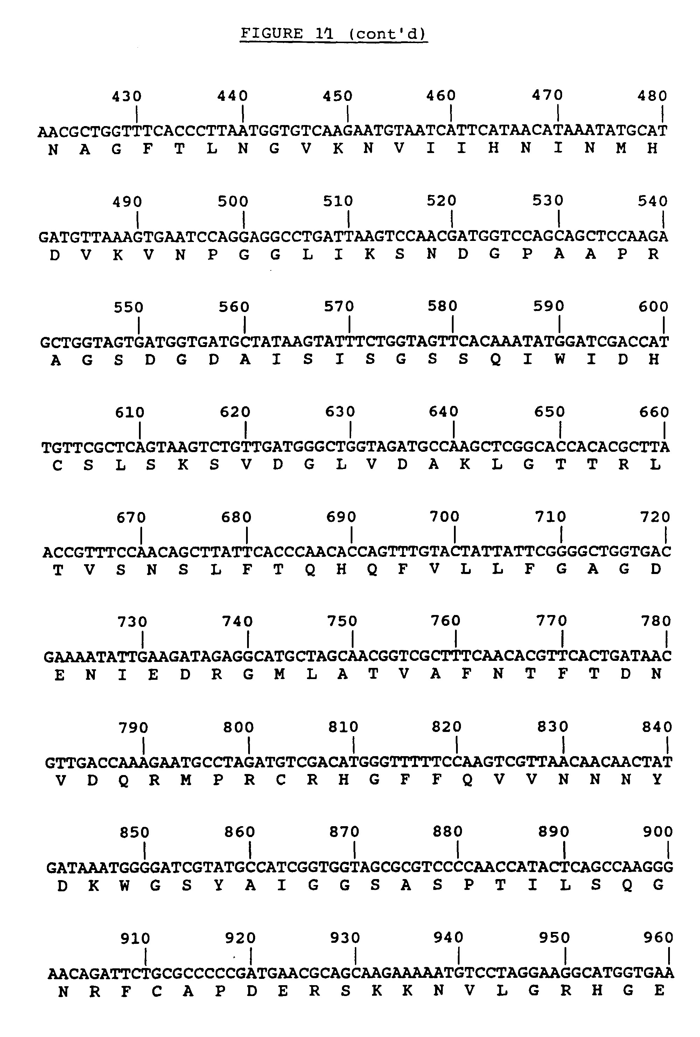

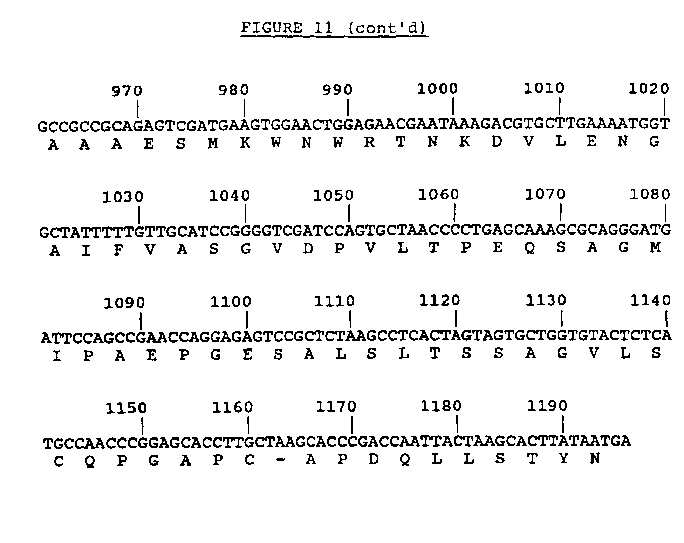

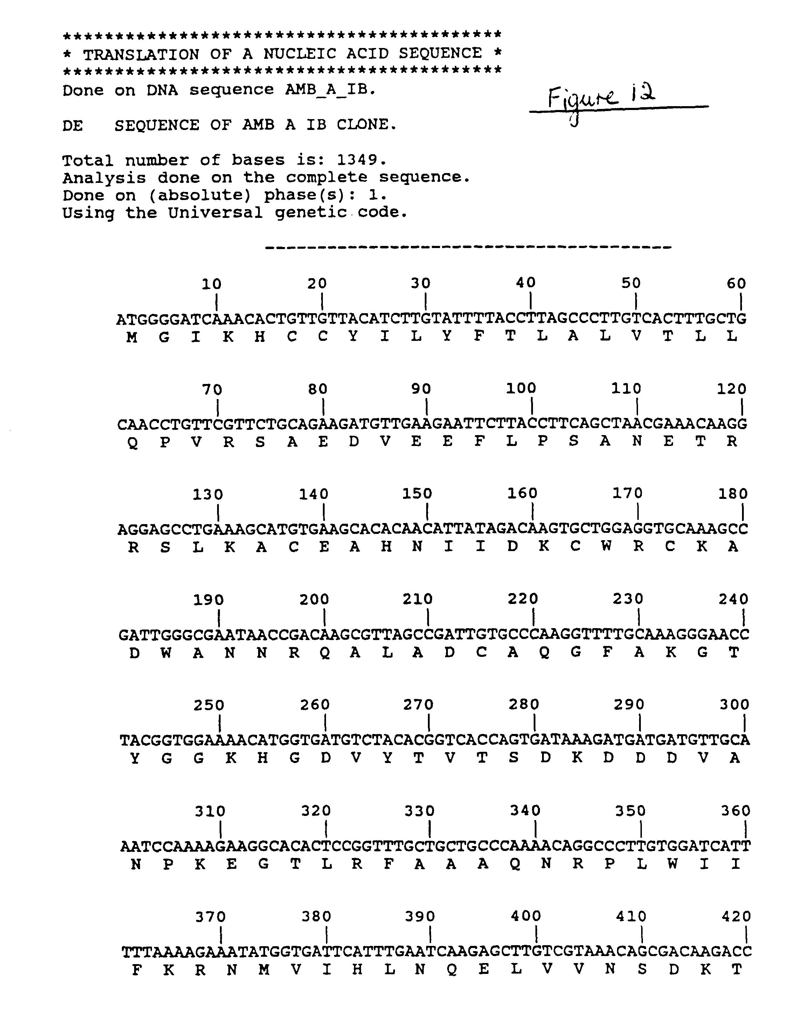

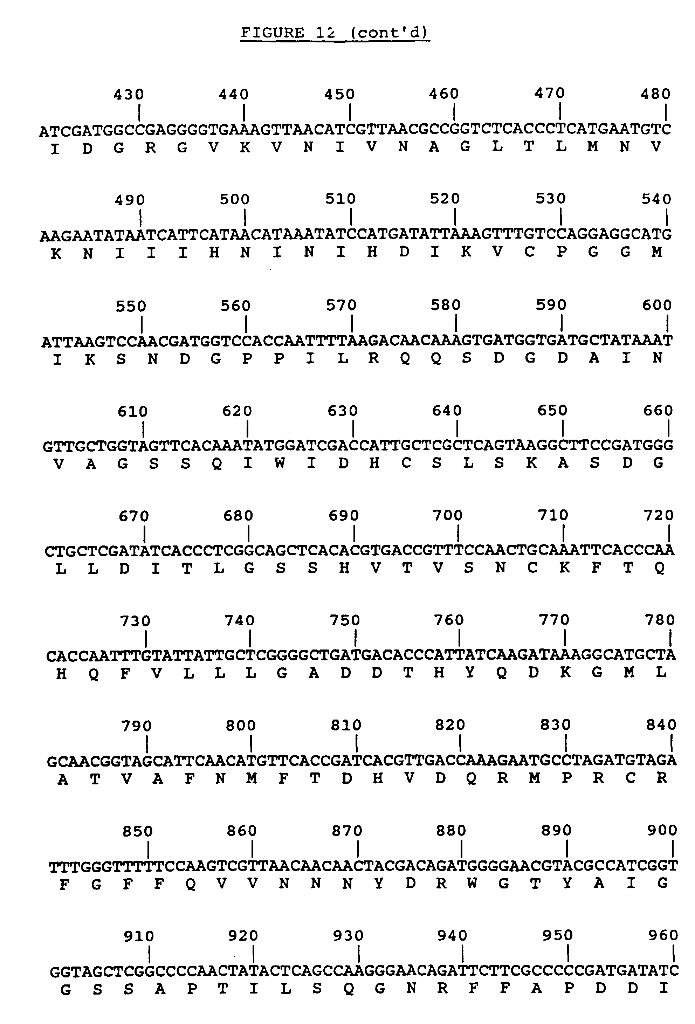

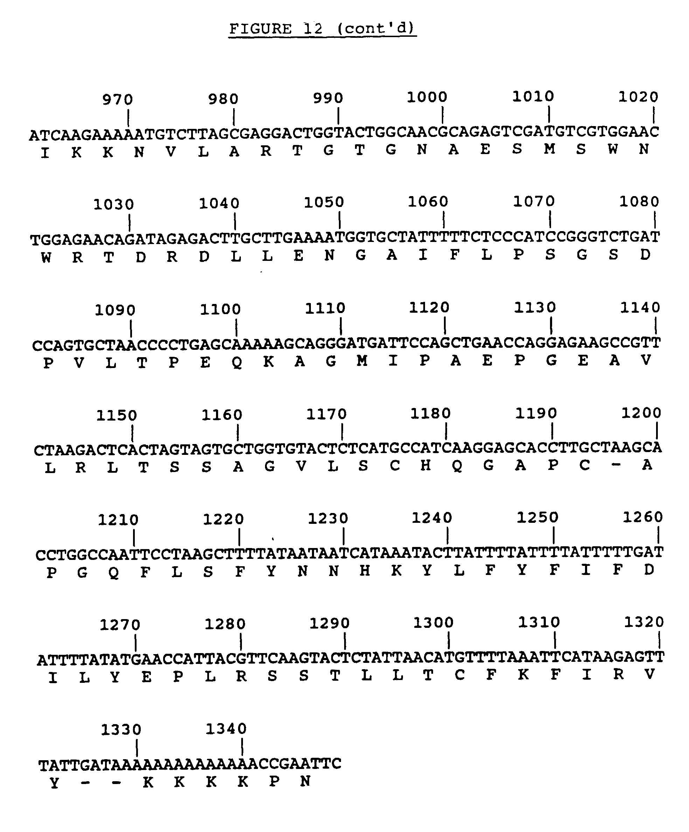

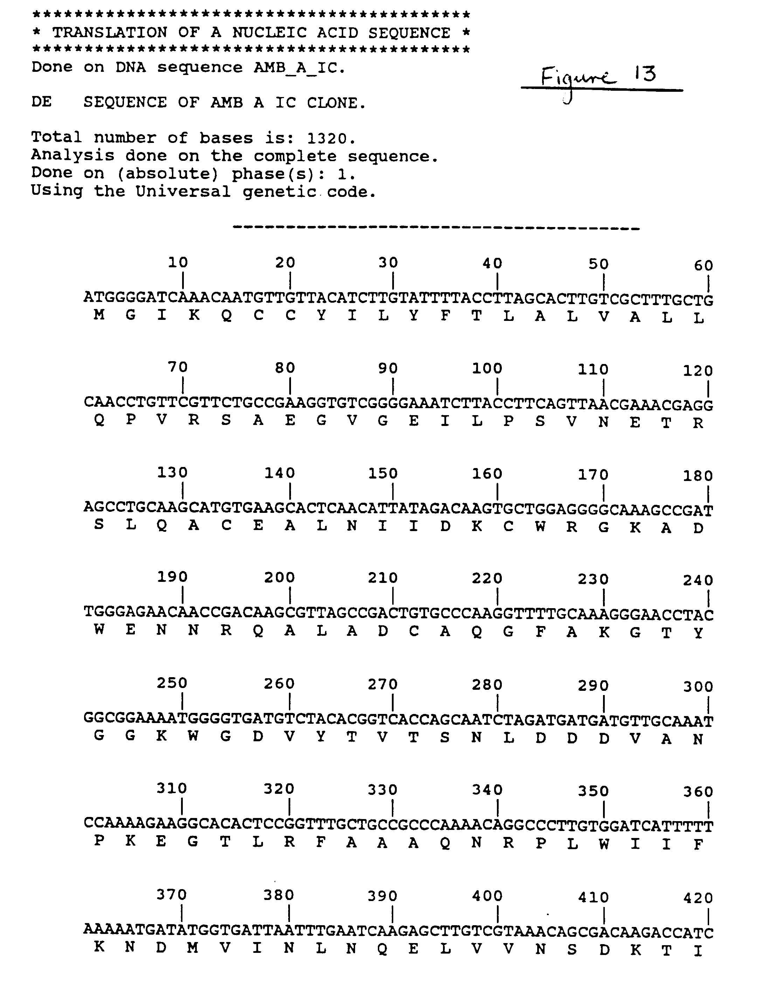

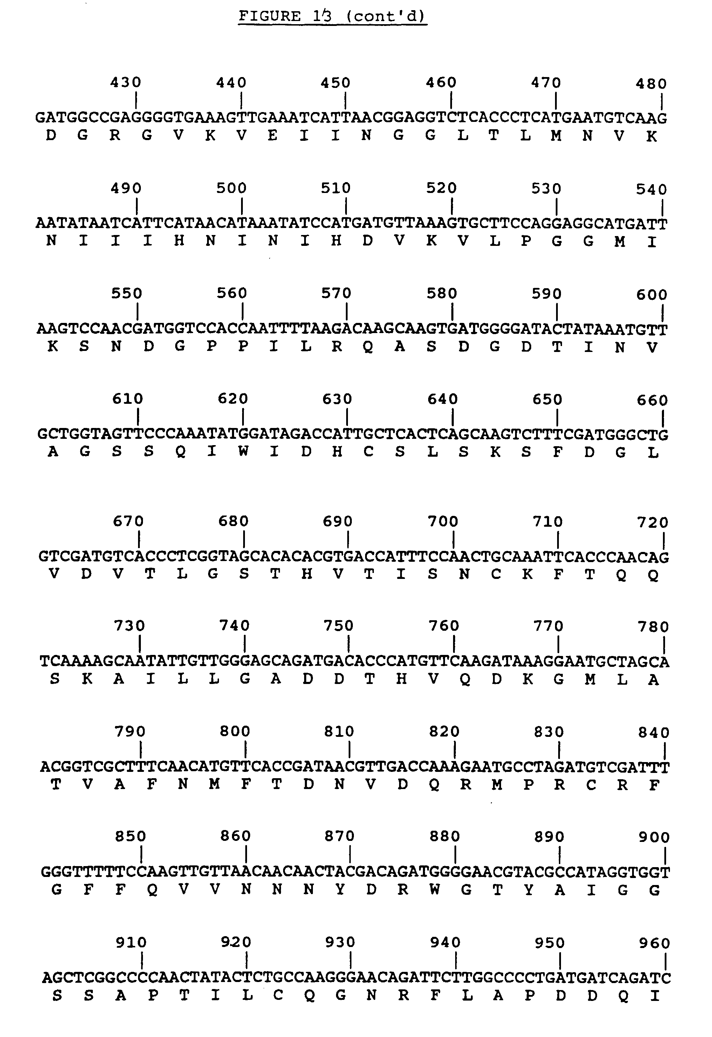

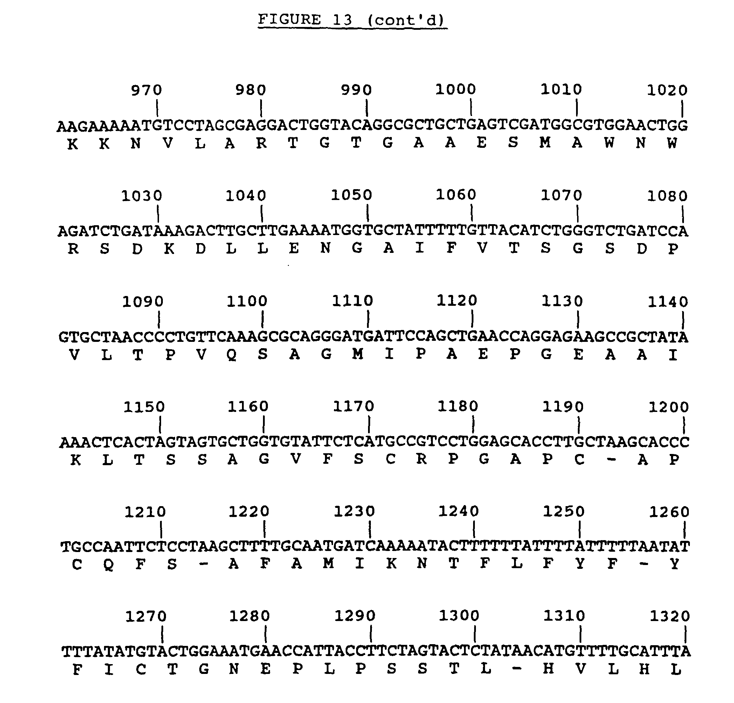

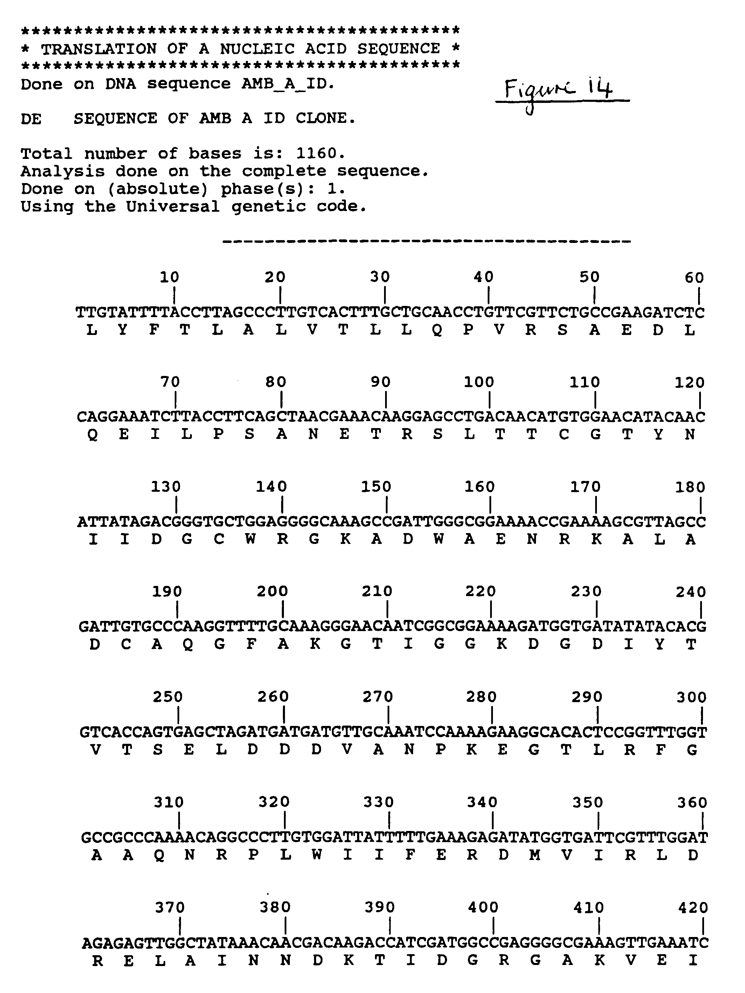

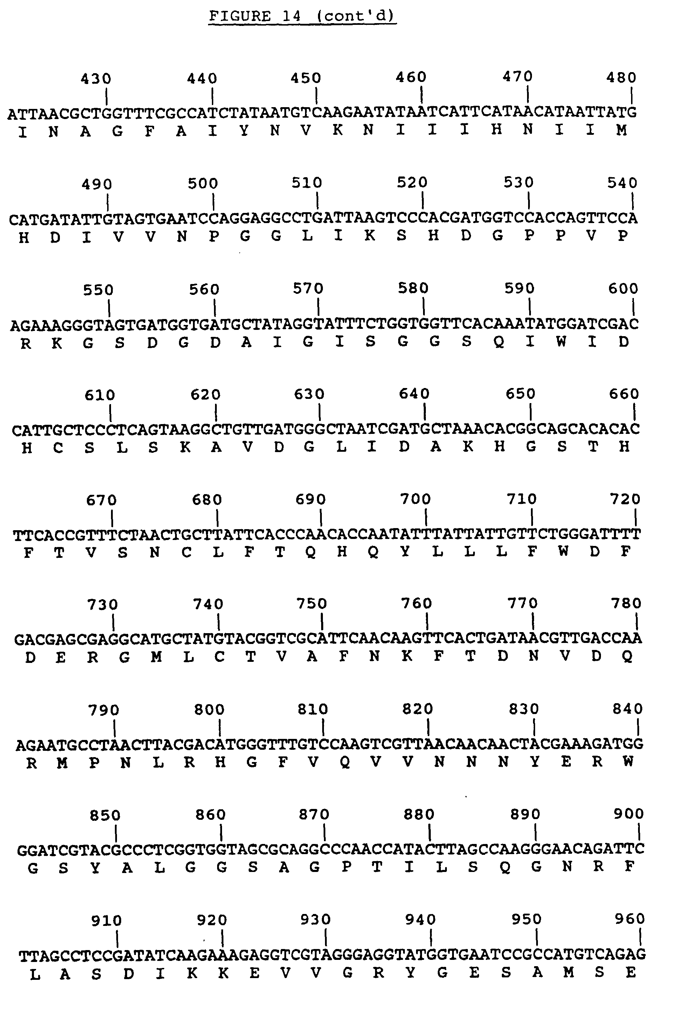

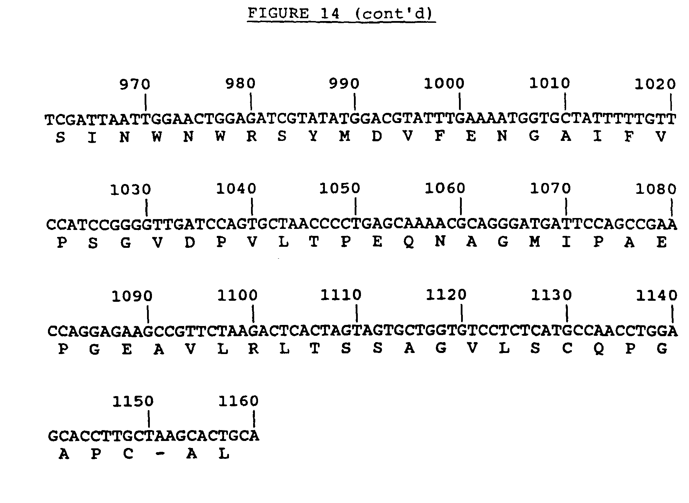

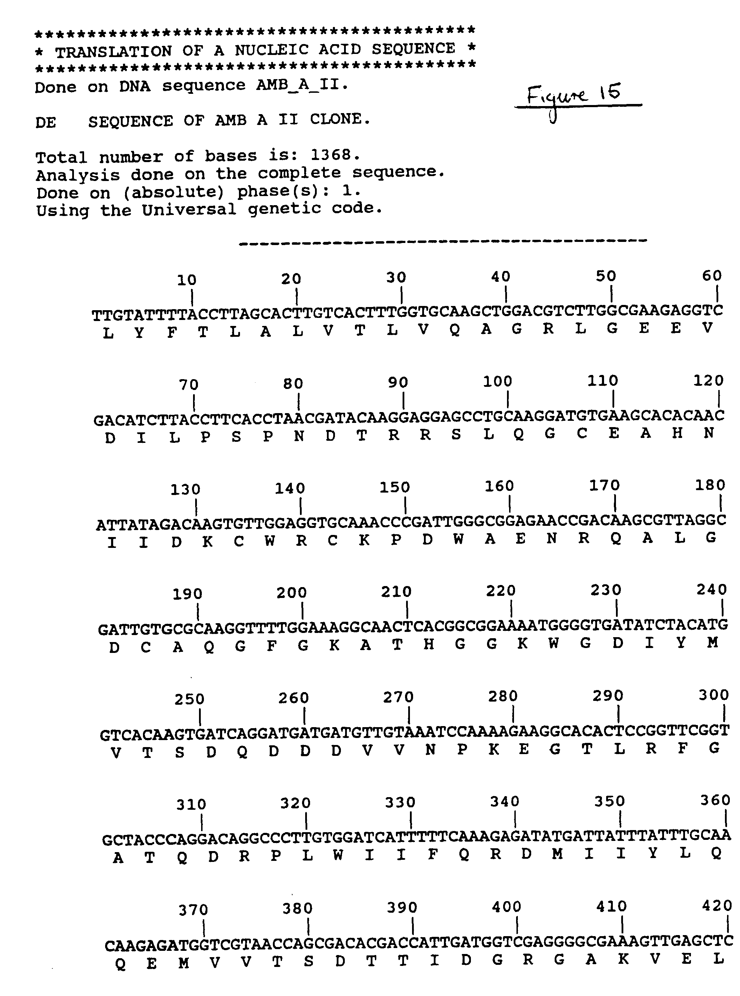

Using the partial cDNA sequences presented in Figures 2-4, cross-hybridization (as described in Example 2) and PCR methods (as described in Example 3) were used to isolate full-length cDNAs encoding Amb a IA (Figure 11), Amb a IB (Figure 12), Amb a IC (Figure 13) and Amb a ID (Figure 14).

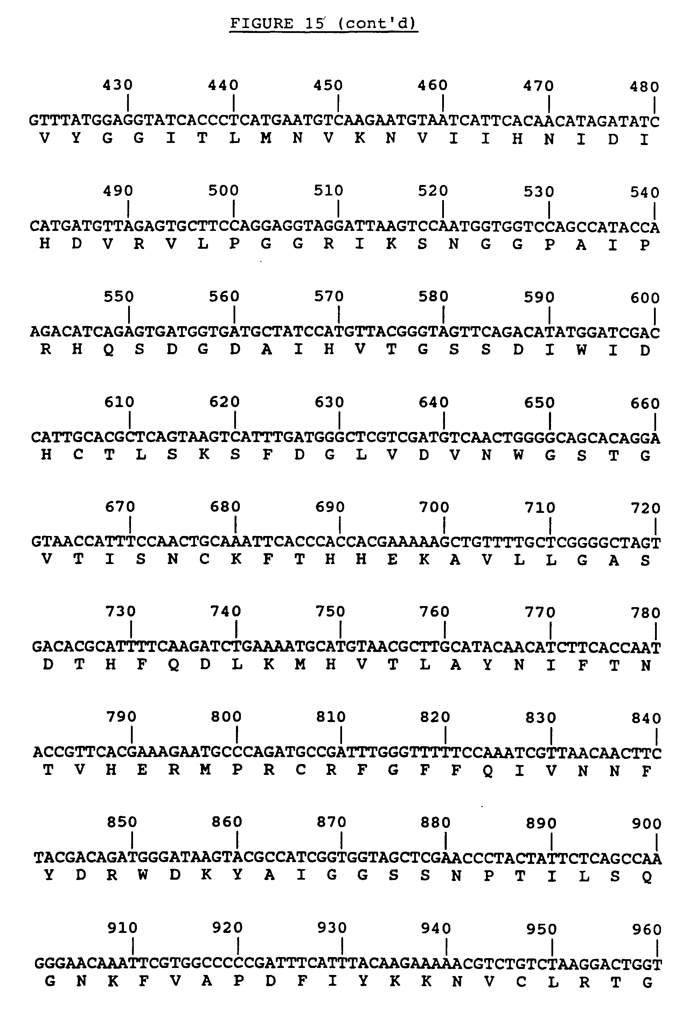

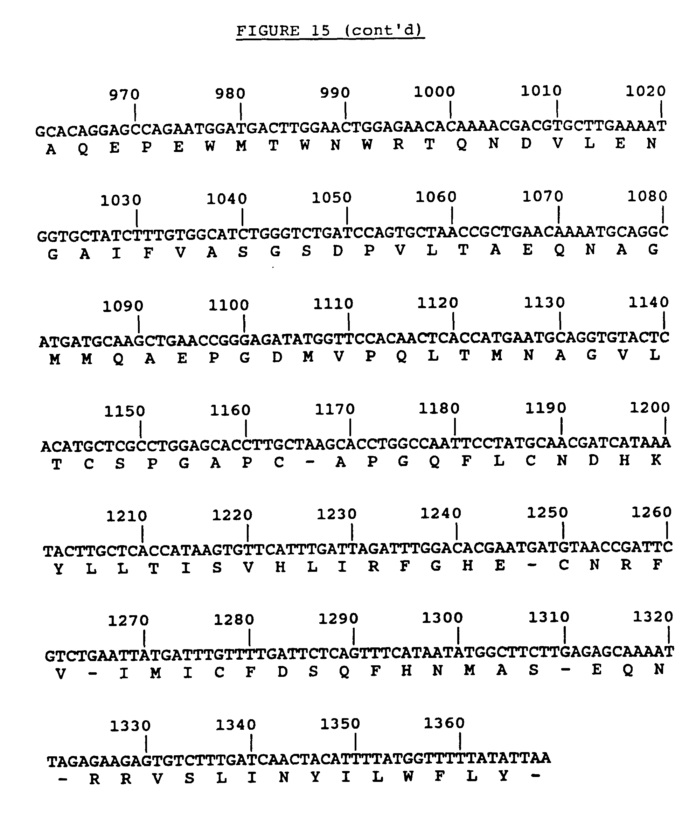

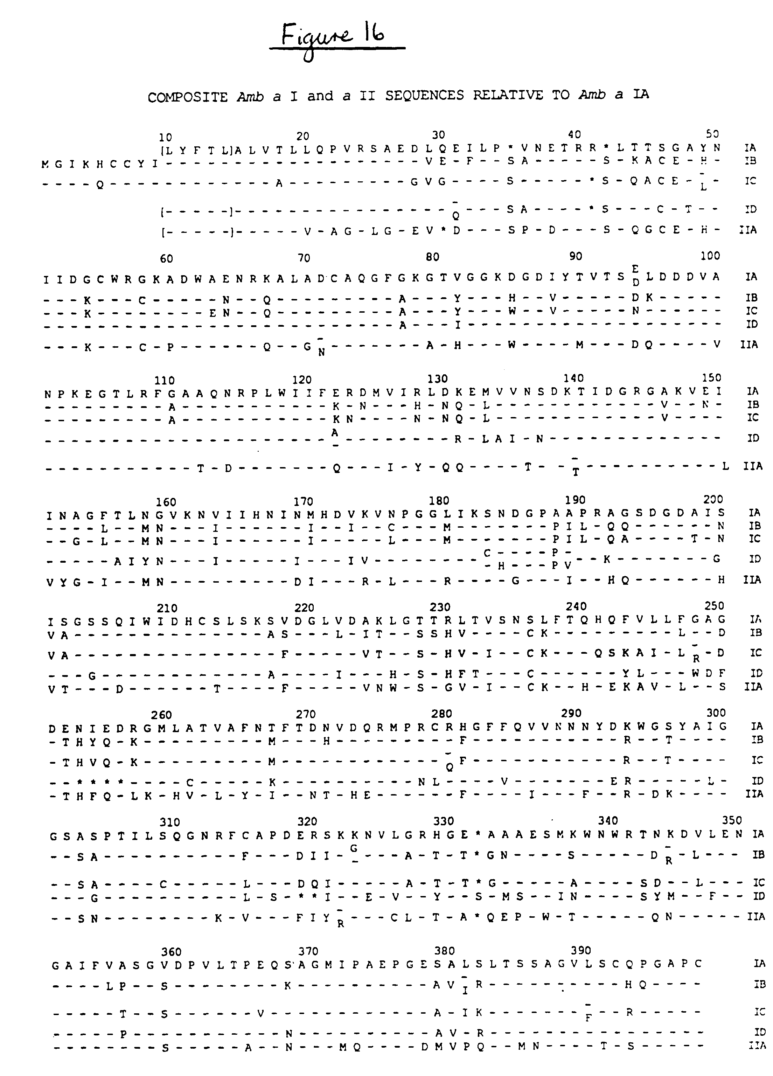

In the course of DNA sequencing of cross-hybridizing cDNAs from a separately constructed λgt10 ragweed flowerhead library, a new cDNA was derived that shared sequence with Amb a II peptide sequence (Figure 15 and Figure 16). Construction of this library and isolation of the new cDNA are described in Example 2. The composite amino acid sequences of the Amb a I and Amb a II multigene family are shown in Figure 16, with the regions of similarity and of disagreement represented. In Figure 16, the sequence of Amb a I is given in standard one-letter code. Sequences for the other Amb a I family members are given relative to that of Amb a I, with only differences being shown. A dash indicates identity between the two sequences. An asterisk indicates a break in the sequence introduced to maintain maximal alignment. Amino acid numbering is based on the Amb a IB sequence. Wherever sequence polymorphism has been observed in a given family member, the dominant sequence is given in superscript and the minor sequence is given in subscript. Polymorphisms in a given family member occur as independent events, except for amino acids 183-189 of Amb a ID, in which the polymorphism occurs as a block.

### Use of an Oligonucleotide Probe to Identify Clones Containing DNA Inserts Encoding Ragweed Protein

As also represented in Figure 1A, an amino acid sequence (WENFK) in the Amb a I preparation, which was identified and sequenced by conventional techniques, was used to deduce the sequence of an oligonucleotide probe (oligoprobe) encoding the amino acid sequence. The amino acid sequence used to deduce the oligonucleotide sequence was VWVKPWENFK. A portion of that amino acid sequence (WENFK) was used to deduce the sequence of the oligoprobe, designated AGE#1. AGE#1 was used, as described in Example 1, to screen a cDNA library constructed in λgt10 using polyA⁺ enriched RNA from pooled short ragweed flower heads. Screening with this oligoprobe resulted in identification of ten duplicated signals. These duplicated signals (clones) were subjected to a secondary screening with the same AGE #1 oligonucleotide probe. Three of the positives (referred to as secondary positives) were clearly detected in duplicate. The clones (designated IPC Clone 1, IPC Clone 5 and IPC Clone 6) identified in this manner were grown under appropriate conditions and verified as positive, by Southern blot analysis.

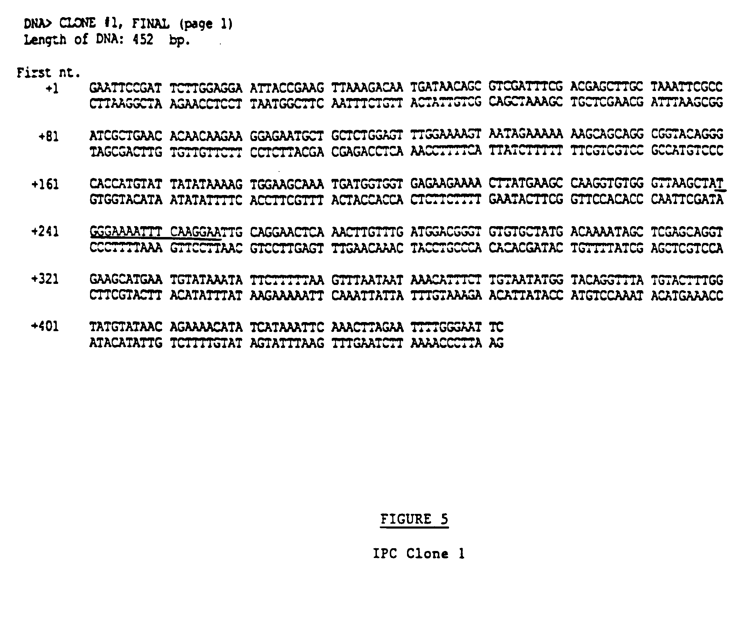

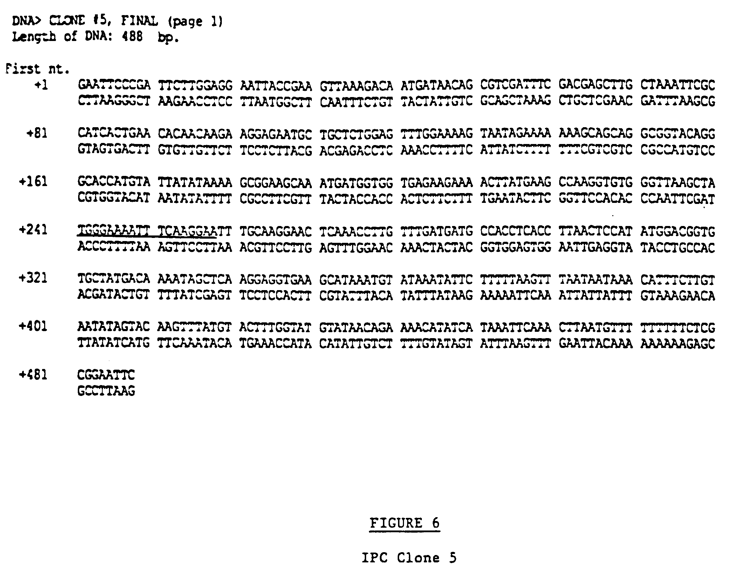

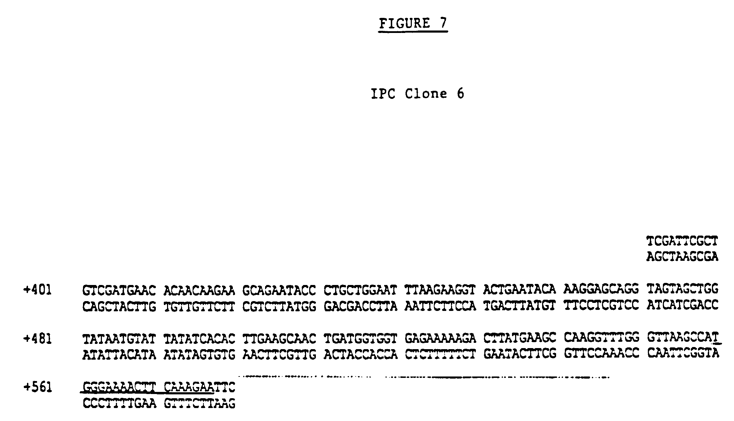

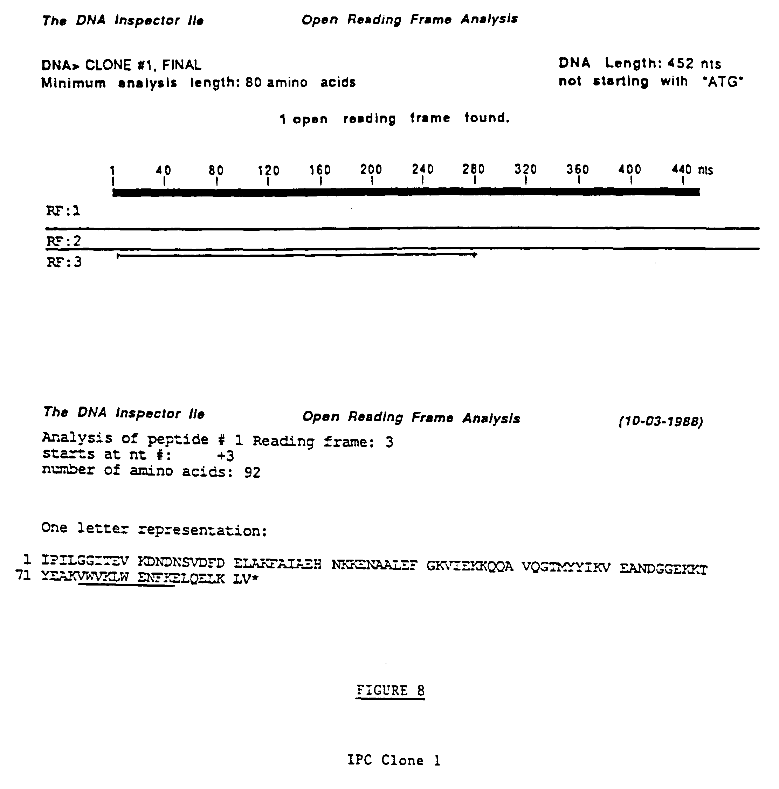

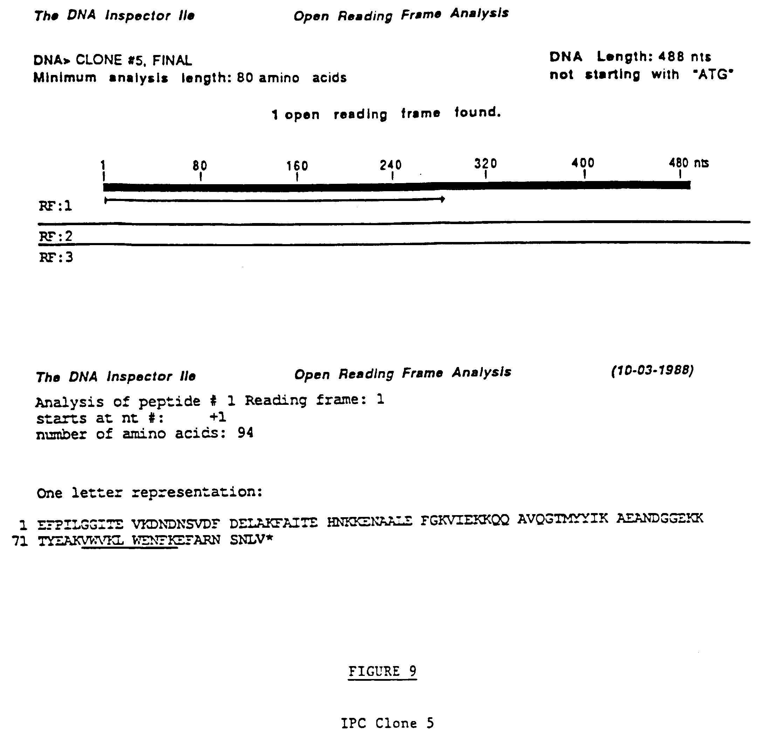

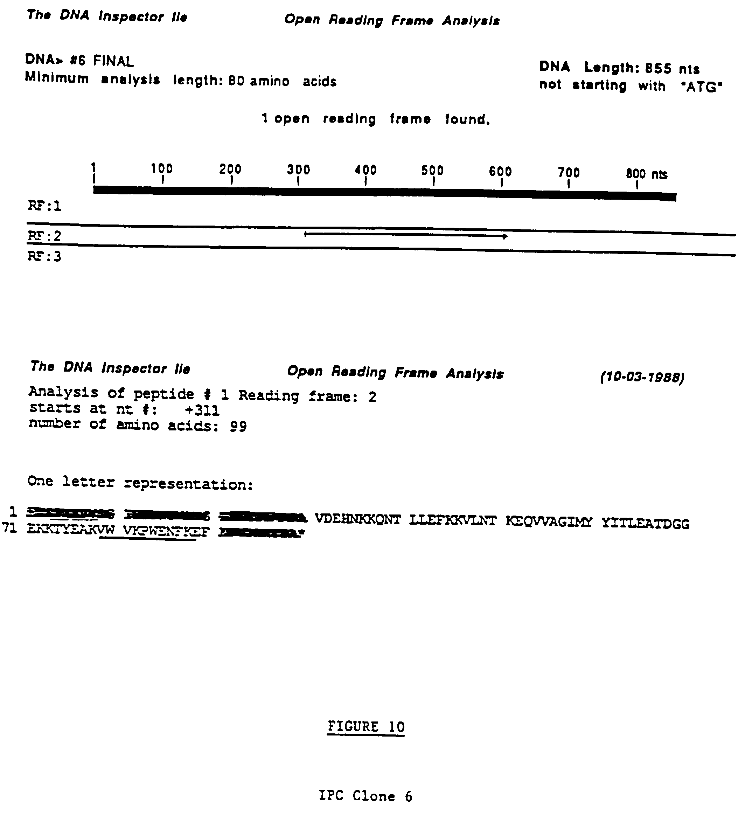

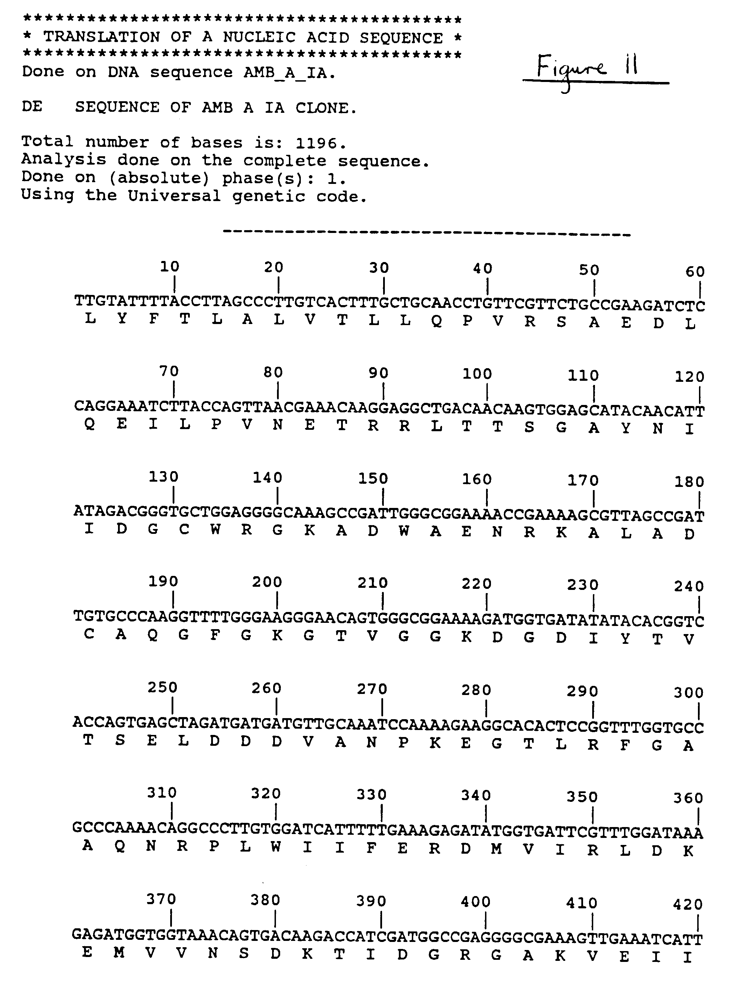

The cDNA insert from each of the three clones was isolated and cloned into M13mp18 and sequenced (Figures 5-7). The amino acid sequence was also deduced (Figures 8-10). Open reading frames in the sequenced cDNAs were examined (Figures 8-10) and the sequence (from which the sequence of the oligonucleotide probe had been deduced) was identified. That the cDNA inserts encode a portion of translated protein was supported by the fact that the surrounding amino acid sequence deduced from the DNA sequence (VWVKP) agreed with the amino acid sequence initially used to deduce the sequence of the oligoprobe (Figure 8-10). T cells from allergic patients could be stimulated by a synthetic peptide RAE4 (Table 5). The RAE4 sequence was deduced from IPC Clone 5 (Figure 8).

As is evident from a comparison of the two "sets" of nucleotide sequences (i.e., set 1, which are the DNAs isolated through use of monoclonal antibodies, and set 2, which are the DNAs isolated through use of the oligoprobe), there is homology among sequences within a set (i.e., within Figures 2-4 and within Figures 5-7) but little similarity in sequences between sets.

Thus, it is apparent that the Amb a I or Antigen E preparation is heterogenous in nature and represents a family (or families) of proteins or that there is considerable polymorphism in Amb a I-encoding DNA. This is in contrast to present literature descriptions of Amb a I or Antigen E, which refer to Antigen E as a protein, rather than as a group or groups of allergenic proteins, present in ragweed pollen, to which ragweed-sensitive individuals respond.

### Additional Demonstration of Isolation of Antigenic Peptides and DNAS of Amb a I

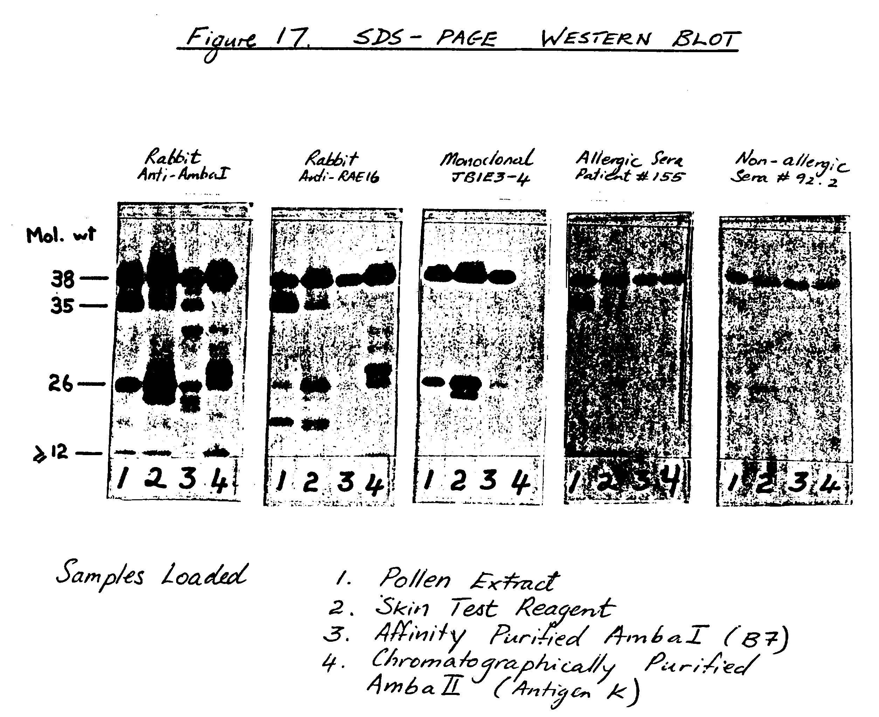

Additional results further demonstrate that antigenic peptides of Amb a I and DNAs encoding them have been identified and isolated. As represented in Figure 1A a selected monoclonal antibody (designated 4B5/B7) which recognizes an Amb a I preparation unsubjected to denaturing conditions was used to affinity purify from pollen extract a single protein, which is referred to as affinity purified Amb a I. This was carried out, using known techniques, by producing the desired monoclonal antibody, isolating it in large quantities from ascites and immobilizing it on Sepharose (Pharmacia). Aqueous pollen extract was passed over the monoclonal antibody-containing column and a protein species was eluted. Antigen E isolated in this manner was shown, using both Western blot (Figure 17) and ELISA techniques, to bind human IgE, thus demonstrating biological activity expected of an Amb a I protein or peptide.

Peptide sequence analysis was carried out as follows: Two peptides were isolated from partial tryptic digestion or cyanogen bromide (CNBr) cleavage of affinity purified Amb a I, respectively, and then subjected to peptide sequencing. Because the N-terminal of Amb a I is blocked, no amino acid sequence can be obtained from direct N-terminal protein sequence analysis. The result of the sequence analysis of the tryptic peptide demonstrated that the major portion of its amino acid sequence agreed with peptide sequence 45 to 77 encoded by the Amb a IA cDNA (Table 1). Table 1 is a comparison of the amino acid sequences of Amb a I protein, determined by protein sequence analysis, with the amino acid sequence deduced from Amb a I cDNA. The CNBr cleavage peptide sequencing demonstrated that the CNBr cleavage peptide was similar to the peptide sequence 171 to 184 encoded by the Amb a IA cDNA (Table 1).

Further peptide sequence analysis was performed from the protein cleavage mixture without isolating individual peptides. The techniques employed involved specific hydrolysis (with 70% formic acid or CNBr) of the putative Asp-Pro and Met-Pro bonds deduced from the cDNA sequences of Amb a I. Any primary amino groups were then blocked by reaction with o-phthalaldehyde prior to conventional sequencing from any available N-terminal proline residue.

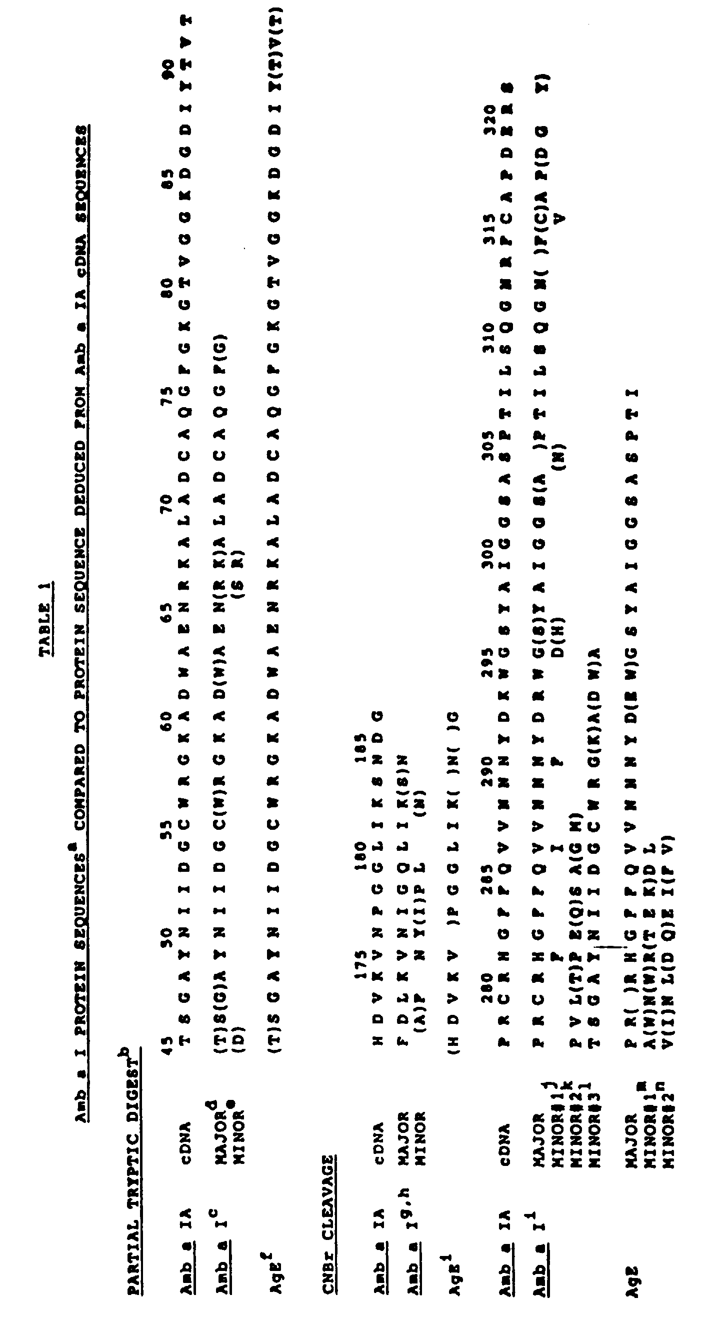

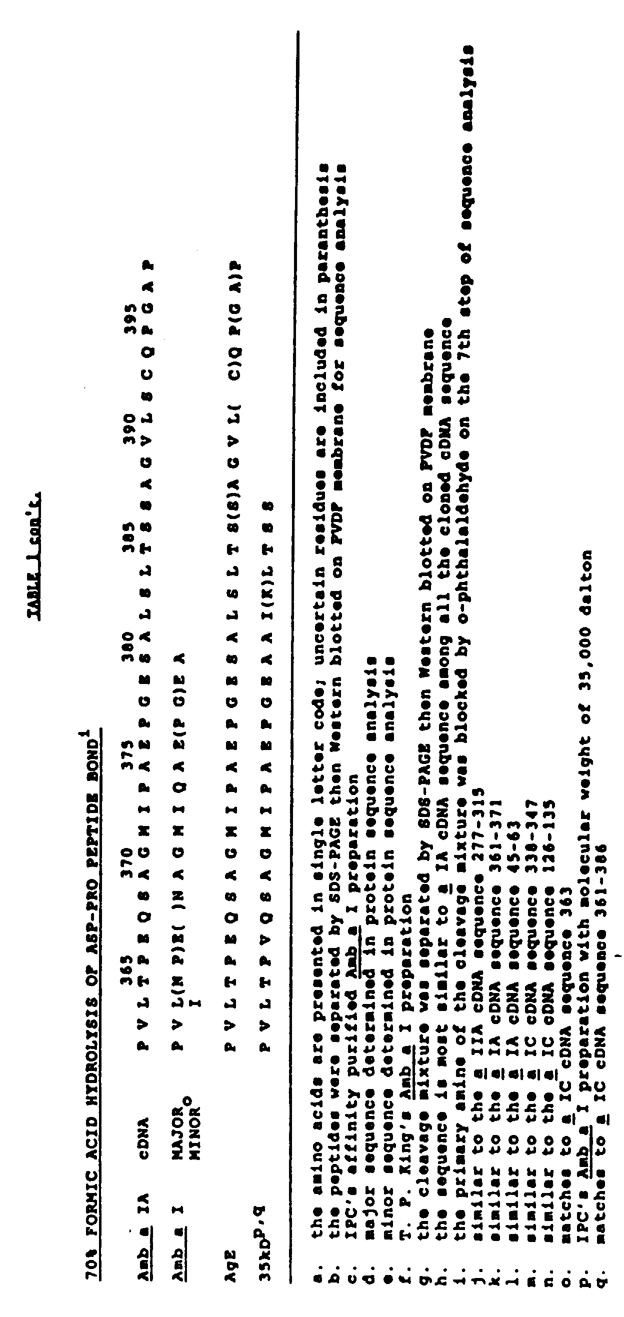

Results of these assessment (shown in Table 1) demonstrated that two peptide sequences determined from the affinity purified Amb a I preparation agreed with that encoded by two portions of Amb a IA DNA sequence (277-321 or 361-397). The minor sequences detected in the peptide sequence analysis also corresponded to a portion of peptide sequence encoded by cDNA's of Amb a I or Amb a II. The above peptide sequence analyses provided strong support that Amb a I or Antigen E-encoding DNA had been isolated.

An Antigen E preparation obtained from Dr. T.P. King was also subjected to peptide sequencing. The same peptide sequencing techniques were employed. Four peptides sequences were identified which agreed with the same four segments of peptide sequence encoded by Amb a IA DNA (45-92, 171-186, 277-321 and 361-397 in Table 1). This provided additional proof that Amb a I or Antigen E-encoding DNA had been isolated.

The same techniques were used with purified Antigen K (Amb a II) from Dr. T.P. King. Results demonstrated that two peptide sequences agreed with two portions of peptide sequence encoded by DNA of Amb a II (Table 2, see Example 2; Figure 15). Table 2 is a comparison of the amino acid sequences of Amb a II protein, determined by protein sequence analysis, with the amino acid sequence deduced from Amb a II cDNA. This finding provided support that ragweed pollen allergen encoding DNA had been isolated.

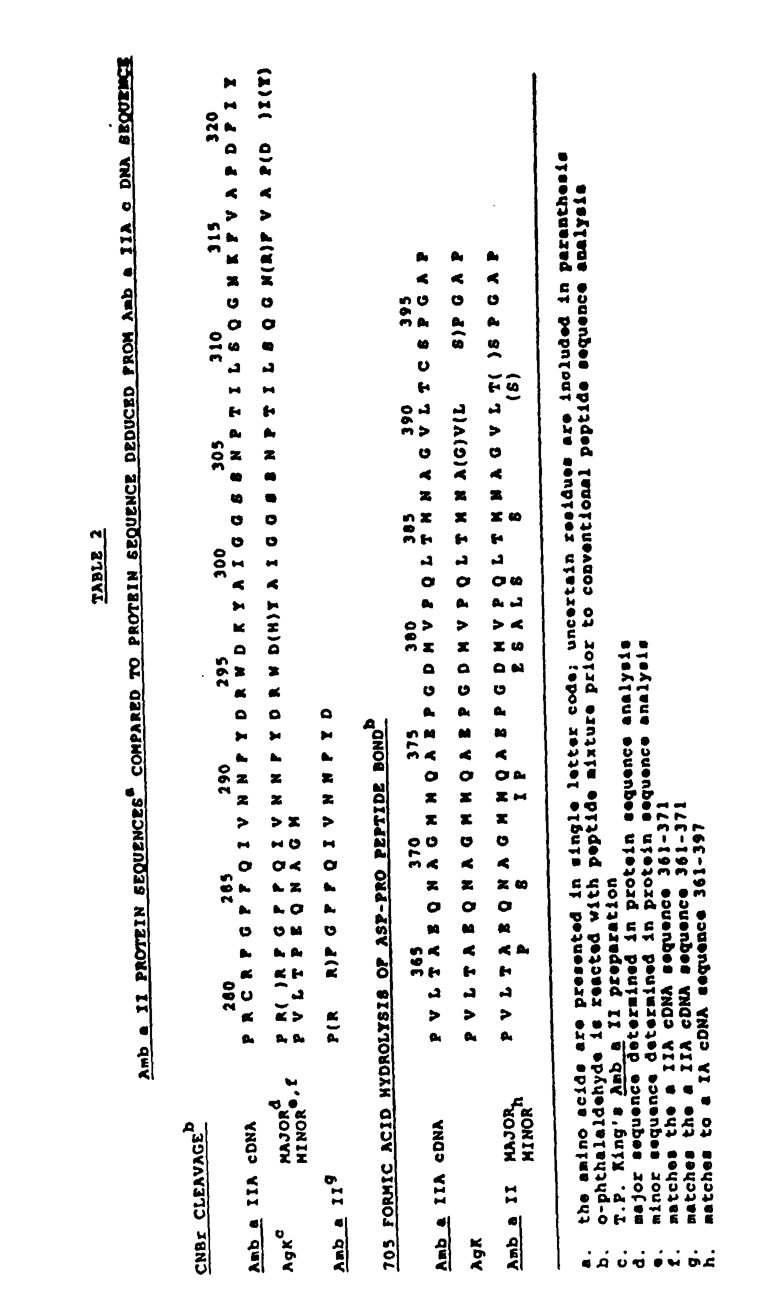

It has been previously reported that Amb a I and Amb a II share some antigenic determinants using rabbit and human antisera (King, T.P., Adv. Immun., 23:77-105 (1976)). However, the exact relationship between the two antigens, until the present invention, has remained unclear. King and colleagues have also reported that different isoforms of antigen E and K (Amb a I and Amb a II) can be isolated by ion-exchange chromatography (King, T.P. et al., Ach. Biochem. Biophys., 212:127-135 (1981)). The different isoforms described, designated A, B, C and D, were interpreted to be produced by limited proteolysis of the intact Amb a I and Amb a II species. It should be noted that these isoforms, designated A, B, C, etc., have no direct relationship with the nomenclature outlined in this invention (i.e., Amb a IA, Amb a IB, etc.).

A 35,000 dalton species coprecipitates from ragweed pollen extract with Amb a II in 45% saturation of ammonium sulfate. Most of these proteins are shown to be agregated by gel filtration chromatography. Some monomeric forms of these proteins were separated from Amb a II by ion exchange chromatography. The sequencing technique, which involved 70% formic acid hydrolysis of putative Asp-Pro bound and o-phthalaldehyde blocking of primary amino groups, demonstrated that the predominant protein corresponds to that encoded by the DNA sequence of Amb a IC. This peptide sequence is referred to as 35kD in Table 1. This result provided additional support that Amb a I proteins are heterogeneous in nature and are encoded by closely related DNA's.

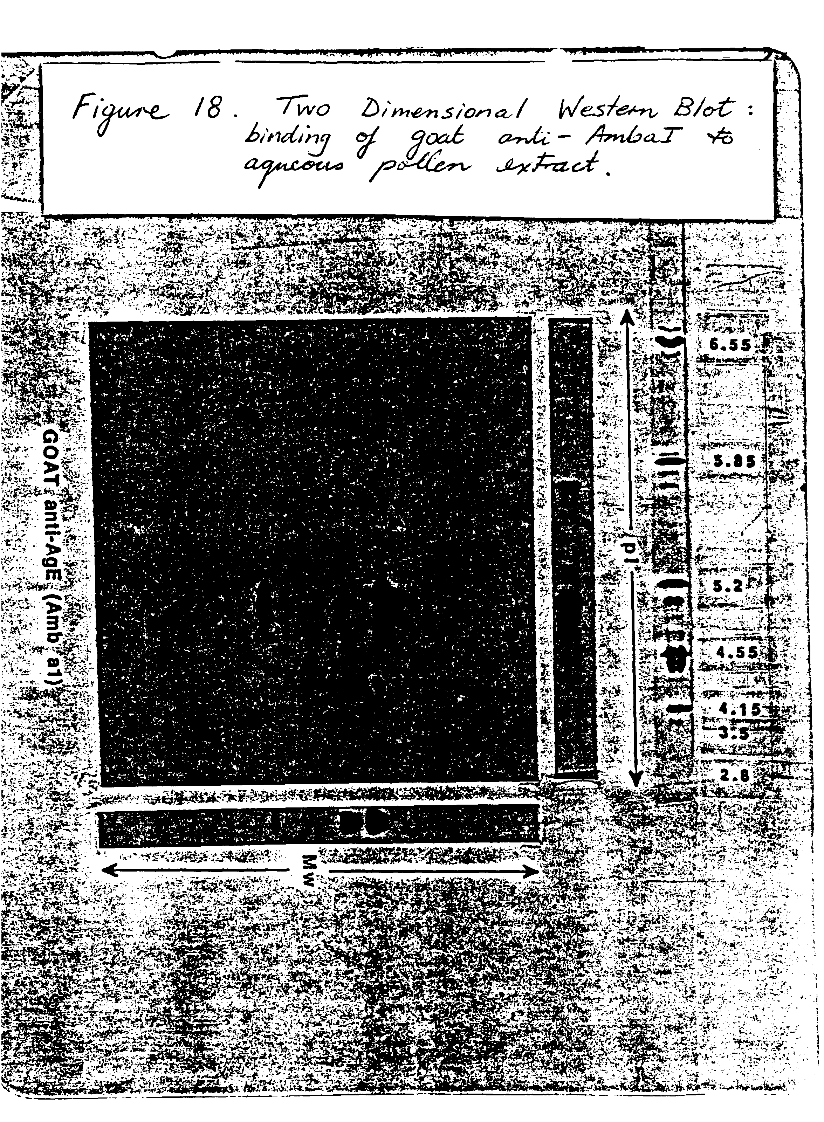

As is also represented in Figure 1A rabbit polyclonal antibodies were produced using the King Antigen E preparation. These antibodies were shown to identify a 38kd protein species on a Western blot of pollen extracts (Figure 17). A two-dimensional gel of ragweed pollen extract, electrophoresed in one dimension on the basis of charge and in the other dimension on the basis of size and treated with goat anti-Amb antibodies is shown in Figure 18. Results demonstrate binding to several proteins present in ragweed pollen extract with a relative molecular weight of 38 kD, corresponding to differently charged forms of what was formerly referred to as Amb a I protein. These antibodies were also shown, using a similar technique, to bind to the affinity purified Amb a I described previously (Figure 17).

It is clear from the antibody reactivity that the 4B5/B7 affinity purified Amb a I has a recognition pattern similar to that of the Amb a I of pollen and skin test reagent with both rabbit polyclonal anti-Amb a I and JB1E3-4 anti-Amb a I monoclonal antibody (Figure 17). It also has readily detectable IgE reactivity on a Western blot (Figure 17; patient number 155). It is also clear that chromatographically purified Amb a II (Antigen K) has cross-reactive B-cell epitopes with the affinity purified Amb a I (Figure 17; anti-Amb a I polyclonal).

As a result of the work described herein, cDNAs encoding allergenic peptides of proteins from a preparation of Amb a I, the major human allergen of ragweed and a preparation of Amb a II, have been cloned, isolated and sequenced; the encoded amino acid sequences (of the allergen(s)) have been deduced and peptides derived from Amb a I and Amb a II have been identified and isolated.

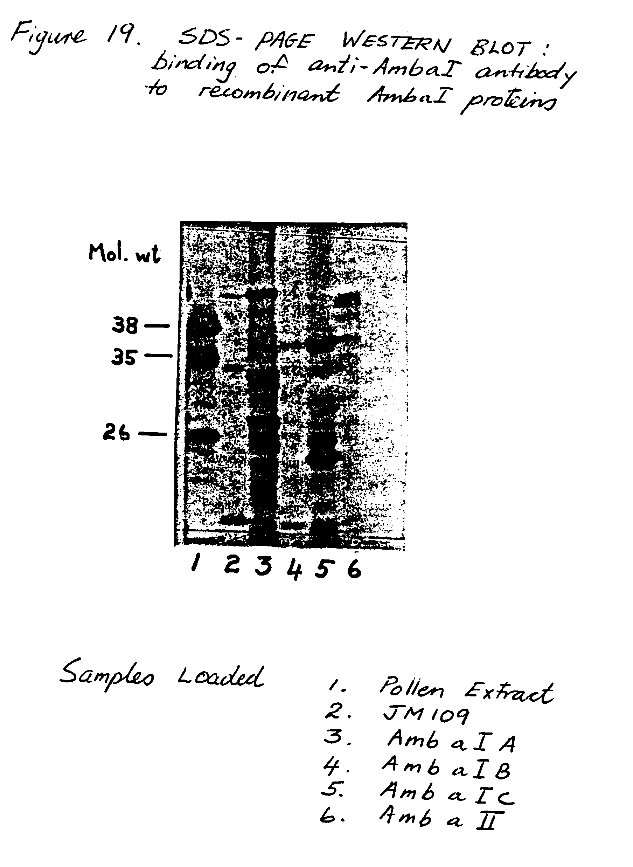

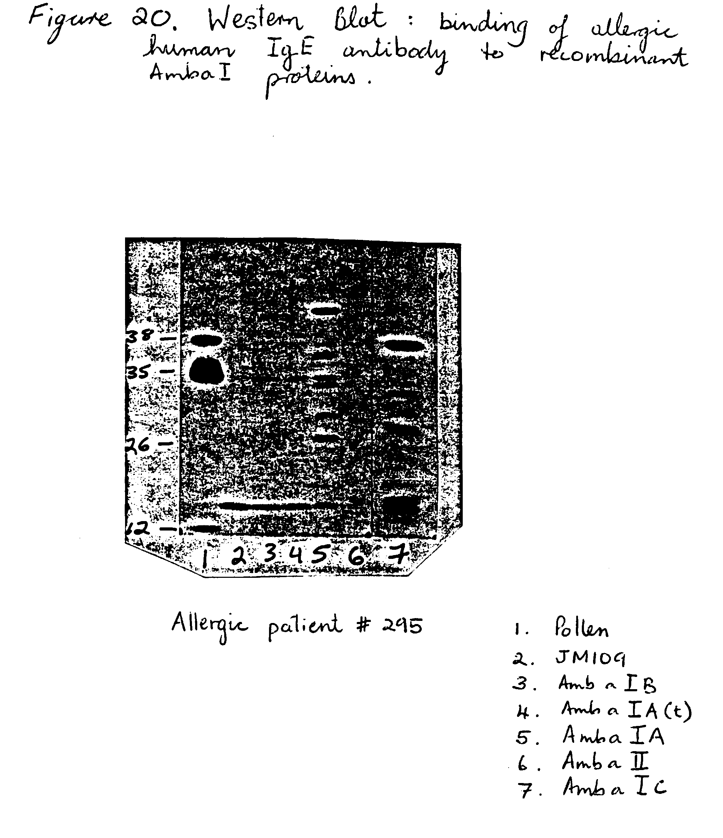

Furthermore, full-length and truncated cDNAs encoding several members of the Amb a I multigene family, as well as Amb a II, were cloned in-frame into the expression vector pTrc99 (Amann et al. Gene, 69: 301-315, (1988)) and transformed into the JM109 host. Expression of recombinant Amb a I and Amb a II protein was induced by 1mM isoprophyl-β-D-thiogalactopyranoside, cells were harvested, lysozyme treated, sonicated and insoluble inclusion bodies recovered by a low speed centrifugation. Recombinant Amb a I and Amb a II protein present in the recovered pellet was solubilized in buffer containing 8M urea, 50mM Tris HCl pH8.0, 50mM NaCl, lmM EDTA, 1mM dithiothreitol, lmM phenylmethylsulfonyl fluoride. After solubilization, the crude urea lysate was dialyzed at 4°C against PBS. The expressed recombinant Amb a I and Amb a II proteins were Western blotted and results are shown in Figures 19 and 20. Results demonstrate (Figure 19) that goat anti-Amb a I antibody binds specifically to several forms of Amb a I (A, B and C), as well as to Amb a II (antigen K). This antigenic cross-reactivity is consistent with the observed sequence homology of the cDNAs (see Figure 16). They further demonstrate (Figure 20) that allergic human IgE binds specifically to some members of the Amb a I multigene family. In the case of patient #295, Amb a IA (full-length) and Amb a IC are bound specifically by IgE to a far greater extent than Amb a IB or Amb a II. A high level of variability in the patterns of IgE binding is seen (Table 3 and data not shown), suggesting that different patients respond to the different Amb a I proteins to different extents.

**TABLE 3**

| SUMMARIZED WESTERN BLOT DATA* | | | | | | |
|---|---|---|---|---|---|---|
| Patient | | Antigen | | | | |
| | Pollen | IA(t) | IA | IB | IC | IIA |
| 151 | + | - | + | - | + | + |
| 222 | +- | +- | +- | +- | +- | + |
| 291 | +++ | + | +++ | - | +++ | + |
| 295 | +++ | + | +++ | + | +++ | - |
| 296 | ++ | | ++ | | ++ | - |

| | | | | | | |
|---|---|---|---|---|---|---|
| - no signal over background +- barely discernable over background + clearly positive ++ strongly positive +++ highly positive * selected from the total of ten patients screened to date. | | | | | | |

An analysis of SDS-PAGE Western blot of IgE binding to several recombinant forms of Amb a I and Amb a II has demonstrated that there is considerable variation in the pattern observed with different patients. Of the ten ragweed allergic patients examined, all possess serum IgE that binds to at least one recombinant Amb a I or Amb a II, with some patient's IgE binding several different recombinant species (summarized in Table 3). Comparison of human IgE binding to recombinant Amb a I and Amb a II proteins with anti-peptide and monoclonal anti-Amb a I antibodies have provided data consistent with the conclusion that the N-terminal portion (historically referred to as the β-region) of Amb a IA includes the major IgE epitope(s). This data (Table 3) is based on the observation that Amb a IA(t) (truncated Amb a IA; amino acid 70-398) binds ragweed allergic patient IgE less well than the full-length Amb a IA (amino acid 10-398). It is expected that the other Amb a I and Amb a II forms possess the same IgE binding properties (see Figure 20, for example).

T cells from patients allergic to ragweed, previously stimulated with a mixed ragweed pollen extract, can recognize and proliferate in response to pollen extract, ragweed skin test reagent (RWST), affinity purified Amb a I protein and crude bacterial lysates containing recombinant Amb a I gene products IA, IB and IC (Table 4). T cells from these patients do not proliferate in the presence of an equivalent amount of control bacterial lysate, JM109. These results demonstrate that each gene product can stimulate some T cell reactivity. The use of crude bacterial lysates as antigens precludes a firm conclusion from the negative responses, since the relative levels of recombinant proteins in lysate have not been determined.

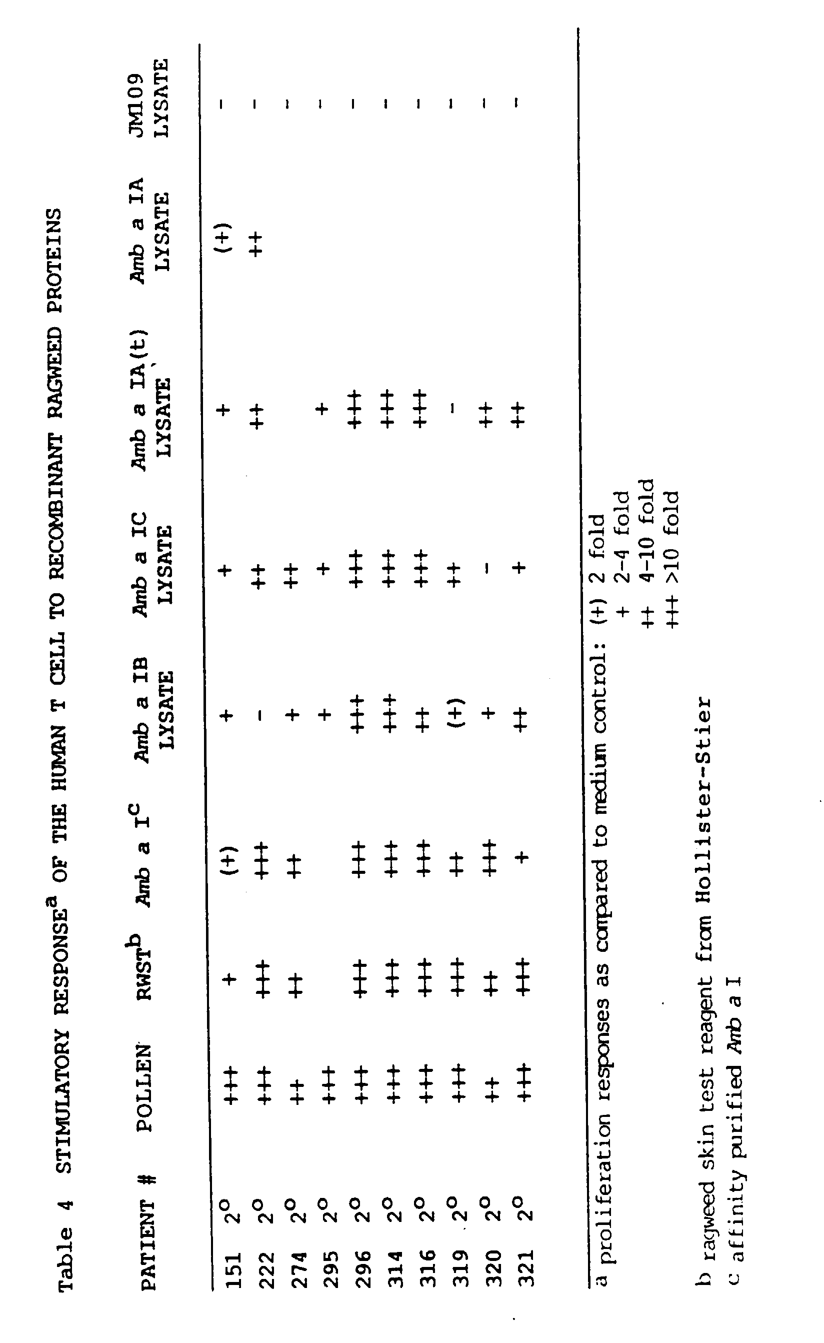

### Uses of the Subject Allerginic Proteins/Peptides and DNA Encoding Same

The materials resulting from the work described herein, as well as compositions containing these materials, can be used in methods of diagnosing, treating and preventing ragweed allergy. In addition, the cDNA (or the mRNA from which it was transcribed) can be used to identify similar sequences in any variety or type of ragweed and, thus, to identify or "pull out" sequences which have sufficient homology to hybridize to, for example, DNA from short ragweed pollen. This can be carried out, for example, under conditions of low stringency; those sequences which have sufficient homology (generally greater than 40%) can be selected for further assessment using the method described herein. Alternatively, high stringency conditions can be used.

In this manner, DNA of the present invention can be used to identify, in other types of ragweed (such as giant ragweed or Western ragweed) sequences encoding peptides having amino acid sequences similar to that of Amb a I and, thus, to identify allergens in such other types of ragweed. Thus, the present invention includes not only Amb a I and other ragweed allergens (e.g., Amb a II or Antigen K) encoded by the present DNA sequences, but also other ragweed allergens encoded by DNA which hybridizes to DNA of the present invention.

Proteins or peptides encoded by the cDNA of the present invention can be used, for example, as "purified" allergens. Such purified allergens are useful in the standardization of allergen extracts which are key reagents for the diagnosis and treatment of ragweed allergy. Furthermore, by using peptides based on the sequences listed in Figures 2 through 16, anti-peptide antisera or monoclonal antibodies can be made using standard methods. Such reagents can be specifically directed against individual isoforms of Amb a I or Amb a II (i.e., directed against divergent regions/epitopes of the molecule) or can be specific for all forms of Amb a I or Amb a II (i.e., directed against common sequences/epitopes). These sera or monoclonal antibodies, directed against Amb a I or Amb a II, can be used to standardize allergen extracts. One such monospecific anti-peptide antisera has already been successfully produced. This rabbit antisera, directed against an Amb a II sequence (amino acid 326-338; designated RAE 50.K with the sequence: CLRTGAQEPEWMT) binds specifically on Western blots to recombinant Amb a II but not Amb a IA, B or C (data not shown).

Through use of the peptides of the present invention, allergen preparations of consistent, well-defined composition and biological activity can be made and administered for therapeutic purposes (e.g., to modify the allergic response of a ragweed-sensitive individual to a ragweed pollen). Such peptides or proteins (or modified versions thereof, such as are described below) may, for example, modify B-cell response to a ragweed allergen, T-cell response to a ragweed allergen or both responses. Purified allergens can also be used to study the mechanism of immunotherapy of ragweed allergy and to design modified derivatives or analogues which are more useful in immunotherapy than are the unmodified ("naturally-occurring") peptides.

Work by others has shown that high doses of allergens generally produce the best results (i.e., best symptom relief). However, many people are unable to tolerate large doses of allergens because of allergic reactions to the allergens. Modification of naturally-occurring allergens can be designed in such a manner that modified peptides or modified allergens which have the same or enhanced therapeutic properties as the corresponding naturally-occurring allergen but have reduced side effects (especially anaphylactic reactions) can be produced. These can be, for example, a peptide of the present invention (e.g., one having all or a portion of the amino acid sequence of a peptide derived from the DNA insert of Clone Amb a IA, Clone Amb a IB, Clone Amb a IC, Amb a II, IPC Clone 1, IPC Clone 5 or IPC Clone 6, or their full-length cDNAs) or a modified peptide or peptide analogue (e.g., a peptide in which the amino acid sequence has been altered to modify immunogenicity and/or reduce allergenicity or to which a component has been added for the same purpose). For example, Amb a I peptides can be modified using the polyethylene glycol method of A. Sehon and co-workers.

Administration of the peptides of the present invention to an individual to be desensitized can be carried out using known techniques. A peptide or combination of different peptides can be administered to an individual in a composition which includes, for example, an appropriate buffer, a carrier and/or an adjuvant. Such compositions will generally be administered by injection, oral administration, inhalation, transdermal application or rectal administration. Using the structural information now available, it is possible to design a ragweed pollen peptide which, when administered to a ragweed-sensitive individual in sufficient quantities, will modify the individual's allergic response to a ragweed allergen. This can be done, for example, by examining the structures of the ragweed proteins, producing peptides to be examined for their ability to influence B-cell and/or T-cell responses in ragweed-sensitive individuals and selecting appropriate epitopes recognized by the cells. Synthetic amino acid sequences which mimic those of the epitopes and which are capable of down regulating allergic response to ragweed allergen can also be used. Proteins, peptides or antibodies of the present invention can also be used for detecting and diagnosing ragweed allergy. For example, by combining blood or blood products obtained from an individual to be assessed for sensitivity to ragweed allergen with an isolated allergenic peptide of ragweed pollen, under conditions appropriate for binding of components (e.g., antibodies, T cells, B cells) in the blood with the peptide and determining the extent to which such binding occurs.

It is now also possible to design an agent or a drug capable of blocking or inhibiting the ability of ragweed allergens to induce an allergic reaction in ragweed-sensitive individuals. Such agents could be designed, for example, in such a manner that they would bind to relevant anti-ragweed IgEs, thus preventing IgE-allergen binding and subsequent mast cell degranulation. Alternatively, such agents could bind to cellular components of the immune system, resulting in suppression or desensitization of the allergic response to ragweed allergens. A non-restrictive example of this is the use of appropriate B- and T-cell epitope peptides, or modifications thereof, based on the cDNA/protein structures of the present invention to suppress the allergic response to ragweed allergens. This can be carried out by defining the structures of B- and T-cell epitope peptides which affect B- and T-cell function in in vitro studies with blood cells from ragweed-sensitive individuals.

The cDNA encoding an allergenic protein or peptide from ragweed can be used to produce additional peptides, using known techniques such as gene cloning. A method of producing a protein or a peptide of the present invention can include, for example, culturing a host cell containing an expression vector which, in turn, contains DNA encoding all or a portion of a selected allergenic protein or peptide (e.g., Amb a I protein or peptide). Cells are cultured under conditions appropriate for expression of the DNA insert (production of the encoded protein or peptide). The expressed product is then recovered, using known techniques. Alternatively, the Amb a I allergen or portion thereof can be synthesized using known mechanical or chemical techniques. As used herein, the term protein or peptide refers to proteins or peptides made by any of these techniques. The resulting peptide can, in turn, be used as described previously.

DNA to be used in any embodiment of this invention can be cDNA obtained as described herein or, alternatively, can be any oligodeoxynucleotide sequence having all or a portion of a sequence represented herein (See Figures 2-16), or their functional equivalents. Such oligodeoxynucleotide sequences can be produced chemically or mechanically, using known techniques. A functional equivalent of an oligonucleotide sequence is one which is capable of hybridizing to a complementary oligonucleotide sequence to which the sequence (or corresponding sequence portions) of Figures 2-16 hybridizes and/or which encodes a product (e.g., a polypeptide or peptide) having the same functional characteristics of the product encoded by the sequence (or corresponding sequence portion) of Figures 2-16. Whether a functional equivalent must meet one or both criteria will depend on its use (e.g., if it is to be used only as an oligoprobe, it need meet only the first criterion and if it is to be used to produce an Amb a I allergen, it need only meet the second criterion).

Antibodies against Amb a I peptides can be used to isolate additional components of ragweed allergens which can be used for further definition of the characteristics of the Amb a I family. Furthermore, anti-peptide sera or monoclonal antibodies directed against Amb a I and/or Amb a II can be used to standardize and define the content of ragweed skin test reagents (RWST). This use would include RWST other than those derived from Ambrosia artemisiifolia I. (e.g., Western, Desert, Giant ragweeds, etc.).

The structural information now available (e.g., DNA, protein/peptide sequences) can also be used to identify or define T cell epitope peptides and/or B cell epitope peptides which are of importance in ragweed allergic reactions and to elucidate the mediators or mechanisms (e.g., interleukin-2, interleukin-4, gamma interferon) by which these reactions occur. This knowledge should make it possible to design peptide-based ragweed therapeutic agents or drugs which can be used to modulate these responses.

The present invention will now be further illustrated by the following Examples, which are not intended to be limiting in any way.

### EXAMPLE 1 Screening of a λgt10 cDNA Library Using an Oligonucleotide Probe

PolyA⁺ enriched RNA extracted from pooled short ragweed flower heads was used to construct a cDNA library in the vector λgt10. The cDNA library was constructed using the Gubler and Hoffman method and the kit supplied by Amersham. Gubler, U. and B.J. Hoffman, Gene, 25:263 (1983). A library of 1.4 x 10⁵ plaques constituting approximately 7 x 10⁴ recombinants was constructed. This library was plated out and screened according to the method of Benton and Davis. Benton, W.D. and R.W. Davis, Science, 196:180 (1977).

### Screening of the CDNA Library:

An amino acid sequence (VWVKPWENFKK), thought to be derived from antigen E was used to deduce an oligonucleotide probe with which to screen the cDNA library.

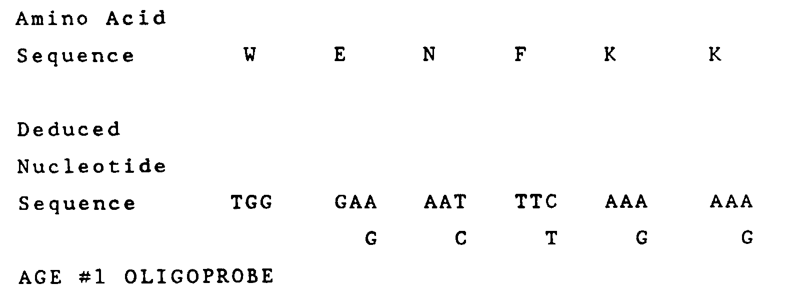

The AGE #1 oligoprobe was end-labeled with ³²P and used to probe 70,000 recombinants using the hybridization conditions listed below:

| | |
|---|---|
| 6 x SSC | |
| 1 x Denhardt's | at 30 degrees C for |
| 50 µg/ml E. coli tRNA | 22 hours |

Ten duplicated signals were detected and these clones were subjected to a secondary screening with the same AGE #1 oligoprobe. It was subsequently discovered that the correct amino acid sequence is WENFKE.
A summary of the cloning procedure is listed below:

### Primary Screen:

70,000 plaques
³²P - AGE #1 oligoprobe
Numerous spots with 10 signals were clearly seen on duplicated filters.

### Secondary Screen:

Plaques from the 10 duplicate signals were picked and plated out at low density and rescreened using methods outlined in Clontech's catalog.

### Tertiary Screening:

Three secondary positives numbered #1, #5, and #6 were clearly detected in duplicate. Each clone was grown up and verified as positive by Southern Blot analysis.

### Sequencing of Positive Clones:

cDNA inserts from each of the clones were isolated then cloned into M13mp18. Each clone was sequenced using the Sanger dideoxy method and the deduced amino acid sequence was determined. Sanger, F. et al., Proc. Natl. Acad. Sci. USA, 74:5463 (1977).

### Identification of WENFKK and Surrounding Sequence:

The DNA sequences of the cDNA clones are presented in Figures 5, 6 and 7. The cDNA clones are not full-length and are less than 500 nucleotides in length. The AGE#1 oligoprobe nucleotide sequence is underlined in Figures 5, 6 and 7. Open reading frames in the sequenced cDNAs were examined and are presented in Figures 8, 9 and 10. The translated amino acid sequence (WENFK) used to deduce AGE#1 oligoprobe sequence is underlined as well as the N-terminal surrounding sequence (VWVKPWENFK; see Figures 8, 9 and 10). IPC clones 1 and 5 disagree with the amino acid sequence at only one out of ten residues (i.e., L instead of P). The presence of the correct surrounding sequence (VWVKP) verifies that the cDNAs encode protein in pollen. Furthermore, a synthetic peptide based on the cDNA sequence designated RAE 4, which has the sequence EFPILGGITEVKDNDNSVDFC, stimulates ragweed allergic patient T cells, in vitro proliferation assays (see Table 5 and sequences in Figures 8, 9 and 10).

### EXAMPLE 2 Cross hybridization Methods Used to Obtain Full-Length cDNAs

Antigen E is reported to be a protein of approximately 38,000 molecular weight and consequently a full-length cDNA encoding this protein must be at least 1.1 Kb in length (King, T.P et al., Arch. Biochem. Biophys., 212:127 (1981)). Consequently, IPC clones 1, 5 and 6 as well as UNC clones 1, 6 and 15 (designated Amb a IA, IB and IC, respectively) are not full-length.

In order to isolate full-length clones, nicktranslated ³²p-labelled Amb a I cDNA probes were used to screen the ragweed flowerhead λgt10 (see Example 1) and the ragweed pollen λgt11 library using standard methods (Maniatis et al., Molecular Cloning, Cold Spring Harbor Laboratory, (1982)). Full-length or near full-length cDNAs encoding Amb a IB (Figures 12 and 16) and Amb a IC (Figures 13 and 16) were isolated using this method (Figure 1B). One cross-hybridizing cDNA clone (called K6-5), which has an open reading frame of approximately 145 amino acids (amino acids 253-398; Figure 15), was found to be significantly divergent from the previously isolated Amb a IA, Amb a IB, Amb a IC and Amb a ID and showed complete agreement (Table 2) with a peptide sequence derived from conventionally purified antigen K (a gift from T.P. King, New York). Consequently, this partial cDNA was designated as Amb a II (see Figure 15 and below).

### EXAMPLE 3 Polymerase Chain Reaction (PCR) Methods Used to Obtain Full-length cDNAs

PCR methods can be successfully used to isolate both rare message cDNA as well as genomic clones of known sequence (Mullis et al., Cold Spring Harbor Symposium Quant. Biol., 51:263-273 (1986)). 5' and 3' oligonucleotide primers were synthesized and used in a PCR experiment with ragweed pollen cDNA serving as template. The 5' primers were deduced from N-terminal conserved regions of Amb a IB (Figure 12) and Amb a IC (Figure 13). The 3' primers were deduced from Amb a IA specific (UNC clone 1, designated Amb a IA, Figure 2) and Amb a II specific (clone K6-5, partial 3' sequence of Figure 15) non-coding strand sequences at the 3' end of the cDNA. A third 3' primer used to PCR clone Amb a ID was derived from a conserved region of the C-terminal end of Amb a IA, B and C (amino-acids 395-398 corresponding to GAPC.stop). The oligonucleotide primers used to amplify and clone Amb a IA, Amb a ID and Amb a II cDNAs are listed below:
N-terminal primers used to produce full-length Amb a IA and Amb a II (amino acids 10-15)

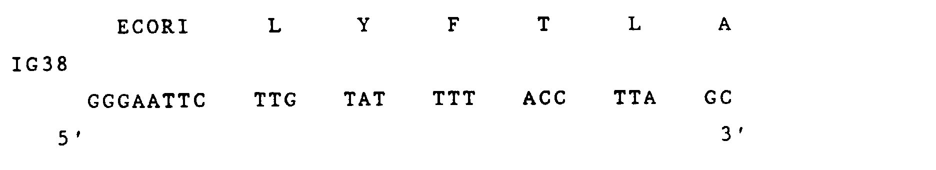

N-terminal primer used to produce truncated Amb a IA and Amb a II (amino acids 70-75)

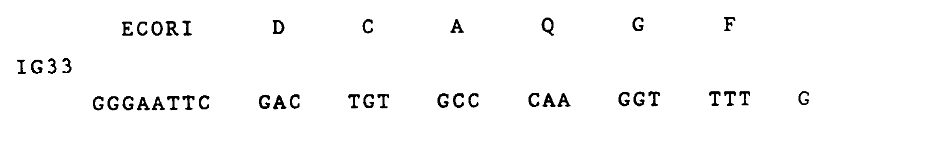

C-terminal primer used to produce full-length and truncated Amb a IA (12-29 nucleotides of the noncoding strand 3' of the TAA stop codon; see Figure 2). 
Pst I
IG32
GGGCTGCAG   TCATTATAAGTGCTTAGT
5'                                                    3'
 C-terminal primer used to produce full-length Amb a ID (corresponding to the C-terminal conserved GAPC encoding region). The primer is of the non-coding strand and includes the stop codon and an artifically introduced Pst I cloning site (see Figure 15). 
Pst I
IG49
GGGCTGCAG TGC TTA GCA AGG TGC TCC
5'                                                    3'
 C-terminal primer used to produce full-length and truncated Amb a II (44-76 nucleotides of the noncoding strand 3' of the TAA stop codon; see Figure 15).

Pst I
AgK2
GGGCTGCAG CGT GTC CAA ATC TAA TCA AAT GAA CAC TTA TGG
5' 3'

First strand cDNA was svnthesized from 1 µg RNA with the cDNA synthesis system plus kit (Amersham) using poly dT as primer. This single stranded cDNA was amplified using sets of primers (IG38 plus IG32; IG33 plus IG32; IG38 plus IG49; IG38 plus AgK2; IG33 plus AgK2) according to methods recommended in the GeneAmp kit (US Biochemicals, Cleveland, OH). The samples were amplified with a programmable thermal controller; the first five rounds of amplification consisted of denaturation at 94° for 30 sec., annealing of primers to the template at 45° for 1 min. 30 sec., and chain elongation at 70° for 4 min. The final 20 rounds of amplification consisted of denaturation as above, annealing at 55° for 1 min. 30 sec. and elongation as above. The PCR generated bands corresponding to the predicted size on an analytical gel and DNA sequencing confirmed that the cDNAs corresponded to full-length and truncated Amb a IA and Amb a II (Figures 11 and 15, respectively) and full-length Amb a ID (Figure 14).

The near full-length cDNAs presented in Figures 11 through 15, have their nucleotide sequences numbered such that the first nucleotide is designated number 1. Although some cDNAs start at what is probably the N-terminal methionine (Amb a IB, Figure 12; Amb a IC, Figure 13), some do not (Amb a IA, Figure 11; Amb a ID, Figure 14; Amb a II, Figure 15) . Consequently, since the cDNAs are of different lengths, their nucleotide numbers. do not necessarily correspond from one sequence to another. The universal genetic code is used to deduce the amino acid sequences from the cDNA sequences and the complete amino acid sequence comparisons between the clones are presented in Figure 16. In Figure 16, the amino acids are numbered sequentially from the probably N-terminal methionine (designated number 1) of the Amb a IB sequence.

### EXAMPLE 4 T Cell Response 5 to Ragweed Proteins and Peptides

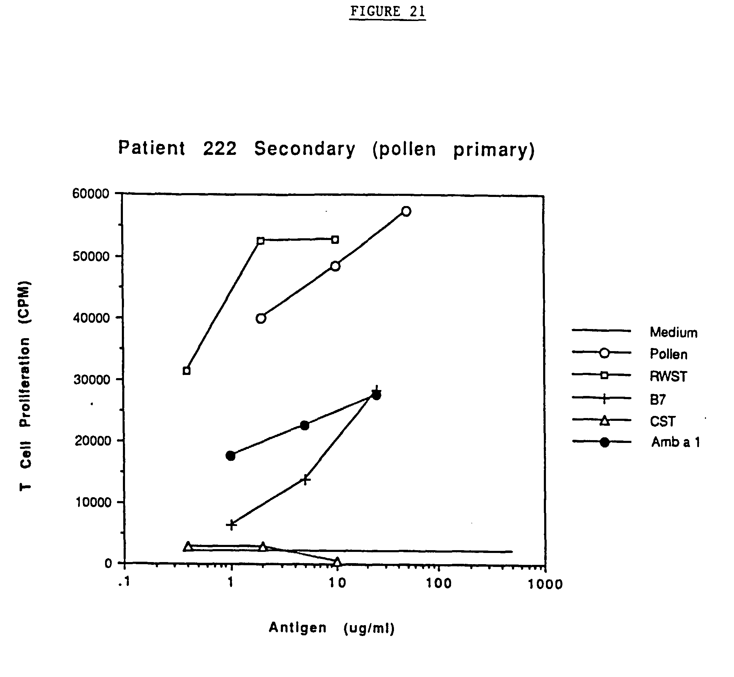

Peripheral blood mononuclear cells (PBMC) were purified from 60 ml of heparinized blood from ragweed-allergic patients. PBMC were subsequently treated as described below, although in individual cases, the length of time of cultivation with IL-2 and IL-4 and the specific ragweed proteins and peptides used for stimulation varied. As an example, ten ml of patient 222 PBMC at 10⁶/ml were cultured at 37°C for 7 days in the presence of 20 micrograms aqueous ragweed pollen extract/ml RPMI-1640 supplemented with 5% pooled human AB serum. Viable cells were purified by Ficoll-Hypaque centrifugation and cultured for three weeks at 5 units recombinant human IL-2/ml and 5 units recombinant human IL-4/ml. The resting T cells were then restimulated (secondary) with 20 micrograms aqueous ragweed pollen extract/ml at a density of 2 x 10⁵ T cells/ml in the presence of X-irradiated (3500 RADS) autologous PBMC (5 x10⁵/ml) for three days, purified by Ficoll-Hypaque centrifugation and grown in 5 units IL-2/ml and 5 units IL-4/ml for two weeks. For assay, 2 x 10⁴ resting secondary T cells were restimulated (tertiary) in the presence of 5 x 10⁴ X-irradiated (3500 RADS) autologous PBMC or 2 x 10⁴ autologous Epstein-Barr virus-transformed B cells (20,000 RADS) with various concentrations of allergen or their fragments in a volume of 200 microliters in 96-well round bottom assay plates for 3 days. Each well then received 1 microCurie tritiated (methyl) thymidine for 16 hours. The counts incorporated were collected onto glass fiber filters and processed for liquid scintillation counting. Figure 21 shows the results of a representative assay, demonstrating the reactivity and specificity of the T cell culture to ragweed pollen proteins. Antigens used: IPC aqueous pollen extract (pollen), Hollister-Stier ragweed skin test extract (RWST), ALK cat epithelium skin test extract (CST), affinity 4B5/B7 antibody purified (dialyzed) Amb a I (B7), and chromatographically purified Amb a I (Amb a I). Medium only control is shown as a line with no symbol. Alternatively, PBMC were sometimes carried only into a secondary assay (as outlined above for a tertiary assay) with 20 micrograms aqueous pollen extract for 7 days, followed by culture in 5 units IL-2/ml and 5 units IL-4/ml for 2-3 weeks. One ragweed allergic patient's T cells in secondary assay responded to pollen extract, RWST, B7 or Amb a I, but did not respond to CST or medium only (Figure 21). Secondary and tertiary assays of a panel of ragweed allergic patients were performed using synthetic peptides derived from the sequences of various ragweed pollen proteins. The results of several experiments are shown in Table 5. Three peptides (RAE16.6, RAE45.15, RAE24.E) which are derived from the sequence of three different Amb a I cDNA's could not stimulate any of the patients' T cells. Another four peptides (RAE15.6, RAE3.D, RAE28.1, RAE26.15) which are also dervied from the sequence of the same three cDNA's could stimulate 35 to 58% of the patients' T cells. One peptide (RAE4) which is derived from the cDNA of IPC Clone 5 could also stimulate 25% of the patients' T cells. These results are consistent with the above cDNA's encoding ragweed pollen proteins. They further demonstrate the opportunity offered by knowledge of the protein structures of the Amb I/II family/ies to identify peptidic fragments which stimulate a response in T cells from ragweed allergic patients and others which do not. By this method it is possible to identify novel therapeutic and diagnostics entities for use in the treatment and the diagnosis of ragweed allergy.

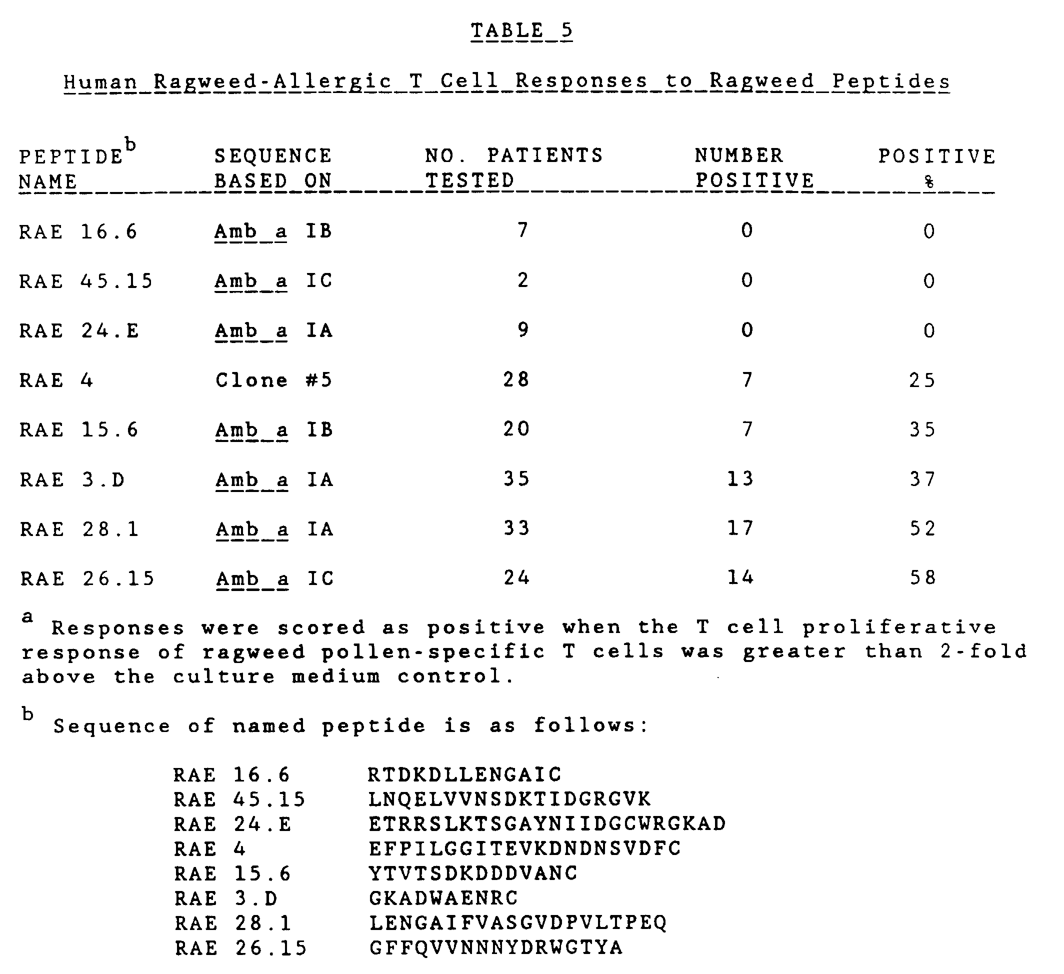

### EXAMPLE 5 Antibody Binding to Recombinant Affinity Purified Amb a I, and Pollen Extract Derived Amb a I and Amb a II

Affinity purified Amb a I was electrophoresed, Western transfered (Towbin et al., Proc. Natl. Acad. Sci. USA, 76:6350 (1979)) and probed with a variety of antibodies, including IgE from an allergic patient (Figure 17). In pollen extract Amb a I is not only present as an intact 38-KD species, but also characterized by its component 26-KD alpha chains and 12-KD beta chains which are formed by enzymatic cleavage. The intact 38-KD species and the alpha chain are clerely detected using rabbit anti-Amb a I, polyclonal affinity purified anti-RAE 16 and monoclonal anti-Amb a I JBIE3-4 (Figure 17; RAE 16 peptide has the sequence RTDKDLLENGAIC derived from amino-acids 342-353 of Amb a IB, Figure 16). Affinity purified Amb a I (partial sequence presented in Table 1) as well as chromatographically purified Amb a II (partial sequence presented in Table 2) are bound on Western blots by these antibodies as well as by patient IgE (Figure 17). The goat anti-Amb a I polyclonal antibody also binds multiple Amb a I and Amb a II species on a two dimensional Western blot of pollen extract (Figure 18). The Western blot was performed as outlined below.

Isoelectric focusing was done on a Hoeffer gel apparatus with 15µg of crude soluble pollen protein. The gel consisted of 7.5% acrylamide with 3.5% Pharmalytes pH 4.5-5.3 (Pharmacia) and 3.5% Ampholines pH 3.5-10 (LKB), run at 13W for 3.5 hours until a constant voltage was reached. The gel section was placed on a slab of 10% acrylamide SDS-PAGE and electrophoresed for 3.5 hours at 40 mA according to the protocol cited. The proteins were transferred overnight in phosphate buffer to 0.1 micron nitrocellulose (Schleicher and Schuell) at 0.2A. The blot was rinsed in blot solution (25mM Tris-HCl pH 7.5, 0.171 M Nacl, 0.05% Tween-20; Sigma). The first antibody incubation was overnight at room temperature with a 1:000 dilution of goat anti-Amb a I IgG (obtained from Dr. David Marsh) in blot solution. The excess first antibody was removed with three 15 minute rinses with blot solution. The second antibody was a 1:2,500 dilution of biotinylated swine anti-goat IgG (Boehringer-Manneheim) in blot solution for two hours. The blot was then rinsed with blot solution three times for 15 minutes and incubated for 1 hr in blot solution with 2µCi I¹²⁵ streptavidin (Amersham). The blots were rinsed with blot solution until the waste wash was down to background. The blot was then exposed to film at -80°C overnight. In the case of one-dimensional SDS-PAGE Western blots (Figures 17, 19 and 20) the isoelectric focusing step was omitted. When human sera was used to probe the Western blots (Figures 17 and 20), 10% human plasma in 1% milk in blot solution was incubated overnight with the blot prior to using as second antibody biotinylated goat anti-human IgE.

## Claims (Claims for the following Contracting State(s): AT, BE, CH, LI, DE, DK, FR, GB, IT, LU, NL, SE)

1. An isolated protein or peptide capable of influencing ragweed B-cell and/or T-cell responses in ragweed-sensitive individuals, the protein or peptide being selected from:
(a) *Amb a* IA free of other *Amb a* I family members and comprising at least a portion of the amino acid sequence shown in Figure 11; or
(b) *Amb a* IB free of other *Amb a* I family members and comprising at least a portion of the amino acid sequence shown in Figure 12; or
(c ) *Amb a* IC free of other *Amb a* I family members and comprising at least a portion of the amino acid sequence shown in Figure 13; or
(d) *Amb a* ID free of other *Amb a* I family members and comprising at least a portion of the amino acid sequence shown in Figure 14, for use in therapy, prophylaxis or diagnosis.

2. A composition capable of influencing B-cell and/or T-cell responses in ragweed-sensitive individuals comprising defined amount(s) of one or more isolated *Amb a* I proteins or peptides, for use in therapy, prophylaxis or diagnosis.

3. The composition of claim 2 which comprises a combination of different *Amb a* I proteins or peptides.

4. An isolated recombinant *Amb a* I protein or peptide for use in therapy, prophylaxis or diagnosis.

5. The protein, peptide or composition of any one of the preceding claims wherein:
(a) the protein or peptide is *Amb a* IA as shown in Figure 11; or
(b) the protein or peptide is *Amb a* IB as shown in Figure 12; or
(c) the protein or peptide is *Amb a* IC as shown in Figure 13; or
(d) the protein or peptide is *Amb* a ID as shown in Figure 14; or
(e) the protein or peptide is a polymorphic variant of any one of (a)-(d).

6. The protein, peptide or composition of any one of the preceding claims wherein the protein or peptide comprisises an amino acid sequence selected from:

a) EFPILGGITEVKDNDNSVDFC;
b) YTVTSDKDDDVANC;
c) GKADWAENRC;
d) LENGAIFVASGVDPVLTPEQ; and
e) GFFQVVNNNYDRWGTYA.

7. The protein, peptide or composition of any one of claims 1 to 4 wherein the *Amb a* I protein or peptide is a giant, desert or western ragweed *Amb a* I variant.

8. The protein, peptide or composition of any one of the preceding claims wherein the protein or peptide is capable of influencing B-cell and/or T-cell responses in ragweed-sensitive individuals and comprises or consists essentially of at least one B cell and/or T cell epitope.

9. The protein, peptide or composition of any one of the preceding claims wherein the protein or peptide has been modified to alter immunogenicity and/or reduce allergenicity.

10. A process for producing a ragweed protein/peptide allergen comprising the steps of:
(a) identifying a DNA sequence encoding the ragweed allergen which hybridizes (for example under high stringency conditions) with the DNA sequence shown in Figure 11, 12, 13 or 14;
(b) inserting the DNA identified in step (a) into an expression vector;
(c) inserting the expression vector of step (b) into a host cell;
(d) culturing the host cell of step (c) under conditions appropriate for expression of the DNA inserted in step (b); and
(e) recovering the expressed product.

11. The process of claim 10 wherein the DNA sequence identified in step (a) has greater than 40% homology with the DNA sequences shown in Figure 11, 12, 13 or 14.

12. Ragweed (for example short, western or giant ragweed) allergen produced by the process of claim 10 or claim 11.

13. A process for preparing a derivative or analogue of a ragweed allergen comprising the steps of:
(a) providing the protein or peptide as defined in any one of claims 1-9 or the allergen of claim 12;
(b) modifying the protein, peptide or allergen of step (a) to produce a derivative or analogue thereof.

14. Ragweed allergen derivative or analogue produced by the process of claim 13.

15. An isolated nucleic acid comprising a nucleotide sequence which encodes an *Amb a* I A, *Amb a* IB, *Amb a* IC or *Amb a* ID protein or peptide.

16. The nucleic acid of claim 15 comprising the coding region shown in Figure 11, 12, 13 or 14.

17. The nucleic acid of claim 16 comprising the nucleotide sequence shown in Figure 11, 12, 13 or 14.

18. A composition comprising the protein or peptide of any one of claims 1 and 4-9 or the allergen of claim 12 or 14 and a buffer, carrier and/or adjuvant.

19. The protein or peptide of any one of claims 1 and 4-9, allergen of claim 12 or 14 or composition of any one of claims 2, 3 and 18 for use in therapy, for example in the treatment for sensitivity in an individual to ragweed pollen.

20. The protein or peptide of any one of claims 1 and 4-9, allergen of claim 12 or 14 or composition of any one of claims 2, 3 and 18 for use in *in vivo* diagnosis, for example in detecting sensitivity in an individual to ragweed pollen.

21. A monoclonal or polyclonal antibody directed against a divergent region/epitope of an *Amb a* I protein or peptide which specifically binds to the *Amb a* IA, *Amb a* IB, *Amb a* IC or *Amb a* ID proteins or peptides defined in any one of tahe preceding claims.

22. A monoclonal or polyclonal antibody directed against a common region/epitope of an *Amb a* I protein or peptide which specifically binds to the *Amb a* IA, *Amb a* IB, *Amb a* IC and *Amb a* ID proteins or peptides defined in any one of the preceding claims.

23. A method of detecting sensitivity in an individual to a ragweed pollen protein/peptide, comprising combining a blood sample of the individual with the protein or peptide of any one of claims 1 and 4-9, allergen of claim 12 or 14 or composition of any one of claims 2, 3 and 18, under conditions appropriate for binding of blood components with the protein or the peptide, and determining the extent to which binding occurs.

24. The method of claim 23, wherein the extent to which binding occurs is determined by assessing T cell function, T cell proliferation, B cell function, binding of the protein or the peptide to antibodies present in the blood, or a combination thereof.

## Claims (Claims for the following Contracting State(s): ES)

1. The isolation of a protein or peptide capable of influencing ragweed B-cell and/or T-cell responses in ragweed-sensitive individuals, the protein or peptide being selected from:
(a) *Amb a* IA free of other *Amb a* I family members and comprising at least a portion of the amino acid sequence shown in Figure 11; or
(b) *Amb a* IS free of other *Amb a* I family members and comprising at least a portion of the amino acid sequence shown in Figure 12; or
(c ) *Amb a* IC free of other *Amb a* I family members and comprising at least a portion of the amino acid sequence shown in Figure 13; or
(d) *Amb a* ID free of other *Amb a* I family members and comprising at least a portion of the amino acid sequence shown in Figure 14, for use in therapy, prophylaxis or diagnosis.

2. A method of preparing a composition capable of influencing B-cell and/or T-cell responses in ragweed-sensitive individuals comprising admixing defined amount(s) of one or more isolated Amb a I proteins or peptides, with an appropriate buffer, carrier and/or adjuvant, for use in therapy, prophylaxis or diagnosis.

3. The method of claim 2 which comprises a combination of different *Amb a* I proteins or peptides.

4. The isolation of a recombinant *Amb a* I protein or peptide for use in therapy, prophylaxis or diagnosis.

5. The isolation or method of any one of the preceding claims wherein:
(a) the protein or peptide is *Amb a* IA as shown in Figure 11; or
(b) the protein or peptide is *Amb a* IB as shown in Figure 12; or
(c) the protein or peptide is *Amb a* IC as shown in Figure 13; or
(d) the protein or peptide is *Amb a* ID as shown in Figure 14; or
(e) the protein or peptide is a polymorphic variant of any one of (a)-(d).

6. The isolation or method of any one of the preceding claims wherein the protein or peptide comprisises an amino acid sequence selected from:

a) EFPILGGITEVKDNDNSVDFC;
b) YTVTSDKDDDVANC;
c) GKADWAENRC;
d) LENGAIFVASGVDPVLTPEQ; and
e) GFFQVVNNNYDRWGTYA.

7. The isolation or method of any one of claims 1 to 4 wherein the *Amb a* I protein or peptide is a giant, desert or western ragweed *Amb a* I variant.

8. The isolation or method of any one of the preceding claims wherein the protein or peptide is capable of influencing B-cell and/or T-cell responses in ragweed-sensitive individuals and comprises or consists essentially of at least one B cell and/or T cell epitope.

9. The isolation or method of any one of the preceding claims wherein the protein or peptide has been modified to alter immunogenicity and/or reduce allergenicity.

10. A process for producing a ragweed protein/peptide allergen comprising the steps of:
(a) identifying a DNA sequence encoding the ragweed allergen which hybridizes (for example under high stringency conditions) with the DNA sequence shown in Figure 11, 12, 13 or 14;
(b) inserting the DNA identified in step (a) into an expression vector;
(c) inserting the expression vector of step (b) into a host cell;
(d) culturing the host cell of step (c) under conditions appropriate for expression of the DNA inserted in step (b); and
(e) recovering the expressed product.

11. The process of claim 10 wherein the DNA sequence identified in step (a) greater than 40% homology with the DNA sequences shown in Figure 11, 12, 13 or 14.

12. A process for preparing a derivative or analogue of a ragweed allergen comprising the steps of:
(a) providing the protein or peptide as defined in any one of claims 1-9 or the allergen of claim 12;
(b) modifying the protein, peptide or allergen of step (a) to produce a derivative or analogue thereof.

13. The isolation of nucleic acid comprising a nucleotide sequence which encodes an *Amb a* IA, *Amb a* IB, *Amb a* IC or *Amb a* ID protein or peptide.

14. The isolation of claim 13 wherein the nucleic acid comprises the coding region shown in Figure 11, 12, 13 or 14.

15. The isolation of claim 14 wherein the nucleic acid comprises the nucleotide sequence shown in Figure 11, 12, 13 or 14.

16. A method of preparing a composition comprising admixing the protein or peptide of any one of claims 1 and 4-9 or an allergen prepared by the process of claim 10 or 12 and a buffer, carrier and/or adjuvant.

17. Use of the protein or peptide of any one of claims 1 and 4-9, allergen prepared by the process of claim 10 or 12 or composition prepared by the method of any one of claims 2, 3 and 16 for the manufacture of a medicament for use in therapy, for example in the treatment for sensitivity in an individual to ragweed pollen.

18. Use of the protein or peptide of any one of claims 1 and 4-9 allergen prepared by the process of claim 10 or 12 or composition prepared by the method of any one of claims 2, 3 and 16 for the manufacture of the medicament for use in *in vivo* diagnosis, for example in detecting sensitivity in an individual to ragweed pollen.

19. The manufacture of a monoclonal or polyclonal antibody directed against a divergent region/epitope of an *Amb a* I protein or peptide which specifically binds to the *Amb a* IA, *amb a* IB, *Amb a* IC or *Amb a* ID proteins or peptides defined in any one of the preceding claims.

20. The manufacture of a monoclonal or polyclonal antibody directed against a common region/epitope of an *Amb a* I protein or peptide which specifically binds to the *Amb a* IA, *Amb a* IB, *Amb a* IC and *Amb a* ID proteins or peptides defined in any one of the preceding claims.

21. A method of detecting sensitivity in an individual to a ragweed pollen protein/peptide, comprising combining a blood sample of the individual with the protein or peptide of any one of claims 1 and 4-9, allergen prepared by the process of claim 10 or 12 or composition prepared by the method of any one of claims 2, 3 and 16, under conditions appropriate for binding of blood components with the protein or the peptide, and determining the extent to which binding occurs.

22. The method of claim 21, wherein the extent to which binding occurs is determined by assessing T cell function, T cell proliferation, B cell function, binding of the protein or the peptide to antibodies present in the blood, or a combination thereof.

## Patentansprüche (Patentansprüche für folgende(n) Vertragsstaat(en): AT, BE, CH, LI, DE, DK, FR, GB, IT, LU, NL, SE)

1. Ein isoliertes Protein oder Peptid mit der Fähigkeit zur Beeinflussung von Ambrosia (ragweed) B-Zell- und/oder T-Zellantworten in Ambrosia-sensitiven Individuen, wobei das Protein oder Peptid ausgewählt ist aus:
(a) *Amb a* IA, frei von anderen *Amb a* I Familienmitgliedern, und das mindestens einen Teil der in Abbildung 11 gezeigten Aminosäure-Sequenz umfaßt; oder
(b) *Amb a* IB, frei von anderen *Amb a* I Familienmitgliedern, und das mindestens einen Teil der in Abbildung 12 gezeigten Aminosäure-Sequenz umfaßt; oder
(c) *Amb a* IC, frei von anderen *Amb a* I Familienmitgliedern, und das mindestens einen Teil der in Abbildung 13 gezeigten Aminosäure-Sequenz umfaßt; oder
(d) *Amb a* ID, frei von anderen *Amb a* I Familienmitgliedern, und das mindestens einen Teil der in Abbildung 14 gezeigten Aminosäure-Sequenz umfaßt, zur Verwendung in Therapie, Prophylaxe oder Diagnose.

2. Eine Zusammensetzung mit der Fähigkeit zur Beeinflussung von B-Zell- und/oder T-Zellantworten in Ambrosia-sensitiven Individuen, die (eine) definierte Menge(n) an einem oder mehreren isolierten *Amb a* I Proteinen oder Peptiden zur Verwendung in Therapie, Prophylaxe oder Diagnose umfaßt.

3. Die Zusammensetzung nach Anspruch 2, die eine Kombination verschiedener *Amb a* I Proteine oder Peptide umfaßt.

4. Ein isoliertes rekombinantes *Amb a* I Protein oder Peptid zur Verwendung in Therapie, Prophylaxe oder Diagnose.

5. Das Protein, Peptid oder die Zusammensetzung nach einem der vorausgehenden Ansprüche, worin:
(a) das Protein oder Peptid *Amb a* IA ist, wie in Abbildung 11 gezeigt; oder
(b) das Protein oder Peptid *Amb a* IB ist, wie in Abbildung 12 gezeigt; oder
(c) das Protein oder Peptid *Amb a* IC ist, wie in Abbildung 13 gezeigt; oder
(d) das Protein oder Peptid *Amb a* ID ist, wie in Abbildung 14 gezeigt; oder
(e) das Protein oder Peptid eine polymorphe Variante eines beliebigen von (a) bis (d) ist.

6. Das Protein, Peptid oder die Zusammensetzung nach einem der vorausgehenden Ansprüche, worin das Protein der Peptid eine Aminosäure-Sequenz umfaßt, die ausgewählt ist aus:

a) EFPILGGITEVKDNDNSVDFC;

b) YTVTSDKDDDVANC;

c) GKADWAENRC;

d) LENGAIFVASGVDPVLTPEQ; und

e) GFFQVVNNNYDRWGTYA.

7. Das Protein, Peptid oder die Zusammensetzung nach einem der Ansprüche 1 bis 4, worin das *Amb a* I Protein oder Peptid eine Ambrosia trifida (giant ragweed), Desert Ragweed oder Ambrosia psilostachya (syn. Ambrosia coronopifolia) (western ragweed) *Amb a* I Variante ist.

8. Das Protein, Peptid oder die Zusammensetzung nach einem der vorausgehenden Ansprüche, worin das Protein oder Peptid die Fähigkeit besitzt, B-Zell- und/oder T-Zellantworten in Ambrosia-sensitiven Individuen zu beeinflussen und im wesentlichen aus mindestens einem B-Zell- und/oder T-Zellepitop besteht oder umfaßt.

9. Das Protein, Peptid oder die Zusammensetzung nach einem der vorausgehenden Ansprüche, worin das Protein oder Peptid zur Änderung der Immunogenität und/oder Reduktion der Allergenität modifiziert worden ist.

10. Ein Verfahren zur Herstellung eines Ambrosia Protein/Peptid-Allergens, das die Schritte umfaßt:
(a) Identifizieren einer das Ambrosia-Allergen codierenden DNS-Sequenz, die (zum Beispiel unter Bedingungen hoher Stringenz) mit der in Abbildung 11, 12, 13 oder 14 gezeigten DNS-Sequenz hybridisiert;
(b) Inserieren der in Schritt (a) identifizierten DNS in einen Expressionsvektor;
(c) Inserieren des Expressionsvektors von Schritt (b) in eine Wirtszelle;
(d) Kultivieren der Wirtszelle von Schritt (c) unter Bedingungen, die für eine Expression der in Schritt (b) inserierten DNS geeignet sind; und
(e) Gewinnen des exprimierten Produkts.

11. Das Verfahren nach Anspruch 10, worin die in Schritt (a) identifizierte DNS-Sequenz eine größere Homologie als 40% mit den in Abbildung 11, 12, 13 oder 14 gezeigten DNS-Sequenz besitzt.

12. Ein Verfahren zur Herstellung eines Derivats oder Analogons eines Ambrosia Allergens, das die Schritte umfaßt:
(a) Bereitstellen des Proteins oder Peptids, wie in einem der Ansprüche 1 bis 9 definiert, oder des Allergens nach Anspruch 12;
(b) Modifizieren des Proteins, Peptids oder Allergens von Schritt (a), um ein Derivat oder Analogon davon herzustellen.

13. Die Isolation einer Nucleinsäure, die eine Nucleotid-Sequenz umfaßt, die ein *Amb a* IA, *Amb a* IB, *Amb a* IC oder *Amb a* ID Protein oder Peptid codiert.

14. Die Isolation nach Anspruch 13, worin die Nucleinsäure die in Abbildung 11, 12, 13 oder 14 gezeigte codierende Region umfaßt.

15. Die Isolation nach Anspruch 14, worin die Nucleinäure die in Abbildung 11, 12, 13 oder 14 gezeigte Nucleotid-Sequenz umfaßt.

16. Die Methode zur Herstellung einer Zusammensetzung, die das Vermischen des Proteins oder Peptids nach einem der Ansprüche 1 und 4 bis 9 oder eines Allergens hergestellt nach dem Verfahren des Anspruchs 10 oder 12 und eines Puffers, Trägers und/oder eines Adjuvans umfaßt.

17. Verwendung des Proteins oder Peptids nach einem der Ansprüche 1 und 4 bis 9, eines Allergens hergestellt nach Anspruch 10 oder 12 oder einer Zusammensetzung hergestellt nach der Methode nach einem der Ansprüche 2, 3 und 16 zur Herstellung eines Medikaments zur therapeutischen Verwendung, zum Beispiel bei der Behandlung einer Sensitivität auf Ambrosia-Pollen in einem Individum.

18. Verwendung des Proteins oder Peptids nach einem der Ansprüche 1 und 4 bis 9, eines Allergens hergestellt nach dem Verfahren nach Anspruch 10 oder 12 oder einer Zusammensetzung hergestellt nach der Methode eines der Ansprüche 2, 3 und 16 zur Herstellung eines Medikaments zur Verwendung in einer *in vivo* Diagnose, zum Beispiel bei Detektion einer Sensitivität auf Ambrosia-Pollen in einem Individuum.

19. Die Herstellung eines monoklonalen oder polyklonalen Antikörpers, der gegen ein(e) divergente(s) Region/Epitop eines *Amb a* I Proteins oder Peptids gerichtet ist und an die in einem der vorausgehenden Ansprüche definierten *Amb a* IA, *Amb a* IB, *Amb a* IC oder *Amb a* ID Proteine oder Peptide spezifisch bindet.

20. Die Herstellung eines monoklonalen oder polyklonalen Antikörpers, der gegen ein(e) gewöhnliche(s) Region/Epitop eines *Amb a* I Proteins oder Peptids gerichtet ist und an die in einem der vorausgehenden Ansprüche definierten *Amb a* IA, *Amb a* IB, *Amb a* IC oder *Amb a* ID Proteine oder Peptide spezifisch bindet.

21. Eine Methode zur Detektion einer Sensitivität auf ein Ambrosia-Pollenprotein/peptid in einem Individuum, die Vereinen einer Blutprobe des Individuums mit dem Protein oder Peptid nach einem der Ansprüche 1 und 4 bis 9, einem Allergen hergestellt nach dem Verfahren nach Anspruch 10 oder 12 oder der Zusammensetzung hergestellt nach einer Methode eines der Ansprüche 2, 3 und 16 unter für ein Binden der Blutkomponenten mit dem Protein oder dem Peptid geeigneten Bedingungen und eine Bestimmung des Ausmaßes, mit dem ein Binden erfolgt, umfaßt.

22. Die Methode nach Anspruch 21, worin das Ausmaß, mit dem ein Binden erfolgt, duch eine Bewertung von T-Zellfunktion, T-Zellproliferation, B-Zellfunktion, Binden des Proteins oder des Peptids an im Blut vorhandene Antikörper oder einer Kombination davon bestimmt wird.

## Patentansprüche (Patentansprüche für folgende(n) Vertragsstaat(en): ES)

1. Die Isolierung eines Proteins oder Peptids mit der Fähigkeit zur Beeinflussung von Ambrosia (ragweed) B-Zell- und/oder T-Zellantworten in Ambrosia-sensitiven Individuen, wobei das Protein oder Peptid ausgewählt ist aus:
(a) *Amb a* IA, frei von anderen *Amb a* I Familienmitgliedern, und das mindestens einen Teil der in Abbildung 11 gezeigten Aminosäure-Sequenz umfaßt; oder
(b) *Amb a* IB, frei von anderen *Amb a* I Familienmitgliedern, und das mindestens einen Teil der in Abbildung 12 gezeigten Aminosäure-Sequenz umfaßt; oder
(c) *Amb a* IC, frei von anderen *Amb a* I Familienmitgliedern, und das mindestens einen Teil der in Abbildung 13 gezeigten Aminosäure-Sequenz umfaßt; oder
(d) *Amb a* ID, frei von anderen *Amb a* I Familienmitgliedern, und das mindestens einen Teil der in Abbildung 14 gezeigten Aminosäure-Sequenz umfaßt, zur Verwendung in Therapie, Prophylaxe oder Diagnose.

2. Eine Methode zur Herstellung einer Zusammensetzung mit der Fähigkeit zur Beeinflussung von B-Zell- und/oder T-Zellantworten in Ambrosia-sensitiven Individuen, die das Vermischen(einer) definierten Menge(n) an einem oder mehreren isolierten *Amb a* I Proteinen oder Peptiden mit einem geeigneten Puffer, Träger und/oder Adjuvans zur Verwendung in Therapie, Prophylaxe oder Diagnose umfaßt.

3. Die Methode nach Anspruch 2, die eine Kombination verschiedener *Amb a* I Proteine oder Peptide umfaßt.

4. Die Isolation eines rekombinanten *Amb a* I Proteins oder Peptids zur Verwendung in Therapie, Prophylaxe oder Diagnose.

5. Die Isolation oder Methode nach einem der vorausgehenden Ansprüche, worin:
(a) das Protein oder Peptid *Amb a* IA ist, wie in Abbildung 11 gezeigt; oder
(b) das Protein oder Peptid *Amb a* IB ist, wie in Abbildung 12 gezeigt; oder
(c) das Protein oder Peptid *Amb a* IC ist, wie in Abbildung 13 gezeigt; oder
(d) das Protein oder Peptid *Amb a* ID ist, wie in Abbildung 14 gezeigt; oder
(e) das Protein oder Peptid eine polymorphe Variante eines beliebigen von (a) bis (d) ist.

6. Die Isolation oder Methode nach einem der vorausgehenden Ansprüche, worin das Protein oder Peptid eine Aminosäure-Sequenz umfaßt, die ausgewählt ist aus:

a) EFPILGGITEVKDNDNSVDFC;

b) YTVTSDKDDDVANC;

c) GKADWAENRC;

d) LENGAIFVASGVDPVLTPEQ; und

e) GFFQVVNNNYDRWGTYA.

7. Die Isolation oder Methode nach einem der Ansprüche 1 bis 4, worin das *Amb a* I Protein oder Peptid eine Ambrosia trifida (giant ragweed), Desert Ragweed oder Ambrosia psilostachya (syn. Ambrosia coronopifolia) (western ragweed) *Amb a* I Variante ist.

8. Die Isolation oder Methode nach einem der vorausgehenden Ansprüche, worin das Protein oder Peptid die Fähigkeit besitzt, B-Zell- und/oder T-Zellantworten in Ambrosia-sensitiven Individuen zu beeinflussen und im wesentlichen aus mindestens einem B-Zell- und/oder T-Zellepitop besteht oder umfaßt.

9. Die Isolation oder Methode nach einem der vorausgehenden Ansprüche, worin das Protein oder Peptid zur Änderung der Immunogenität und/oder Reduktion der Allergenität modifiziert worden ist.

10. Ein Verfahren zur Herstellung eines Ambrosia Protein/Peptid-Allergens, das die Schritte umfaßt:
(a) Identifizieren einer das Ambrosia-Allergen codierenden DNS-Sequenz, die (zum Beispiel unter Bedingungen hoher Stringenz) mit der in Abbildung 11, 12, 13 oder 14 gezeigten DNS-Sequenz hybridisiert;
(b) Inserieren der in Schritt (a) identifizierten DNS in einen Expressionsvektor;
(c) Inserieren des Expressionsvektors von Schritt (b) in eine Wirtszelle;
(d) Kultivieren der Wirtszelle von Schritt (c) unter Bedingungen, die für eine Expression der in Schritt (b) inserierten DNS geeignet sind; und
(e) Gewinnen des exprimierten Produkts.

11. Das Verfahren nach Anspruch 10, worin die in Schritt (a) identifizierte DNS-Sequenz eine größere Homologie als 40% mit den in Abbildung 11, 12, 13 oder 14 gezeigten DNS-Sequenzen besitzt.

12. Ambrosia (zum Beispiel Ambrosia artemisiifolia, Ambrosia psilostachya (syn. Ambrosia coronopifolia) oder Ambrosia trifida) Allergen, das mit dem Verfahren nach Anspruch 10 oder 11 hergestellt ist.

13. Ein Verfahren zur Herstellung eines Derivats oder Analogons eines Ambrosia Allergens, das die Schritte umfaßt:
(a) Bereitstellen des Proteins oder Peptids, wie in einem der Ansprüche 1 bis 9 definiert, oder des Allergens nach Anspruch 12;
(b) Modifizieren des Proteins, Peptids oder Allergens von Schritt (a), um ein Derivat oder Analogon davon herzustellen.

14. Ambrosia Allergen-Derivat oder Analogon, das nach dem Verfahren nach Anspruch 13 hergestellt ist.

15. Eine isolierte Nucleinsäure, die eine Nucleotid-Sequenz umfaßt, die ein *Amb a* IA, *Amb a* IB, *Amb a* IC oder *Amb a* ID Protein oder Peptid codiert.

16. Die Nucleinsäure nach Anspruch 15, die die in Abbildung 11, 12, 13 oder 14 gezeigte codierende Region umfaßt.

17. Die Nucleinsäure nach Anspruch 16, die die in Abbildung 11, 12, 13 oder 14 gezeigte Nucleotid-Sequenz umfaßt.

18. Eine Zusammensetzung, die das Protein oder Peptid nach einem der Ansprüche 1 und 4 bis 9 oder das Allergen nach Anspruch 12 oder 14 und einen Puffer, Träger und/oder ein Adjuvans umfaßt.

19. Das Protein oder Peptid nach einem der Ansprüche 1 und 4 bis 9, das Allergen nach Anspruch 12 oder 14 oder einer Zusammensetzung nach einem der Ansprüche 2, 3 und 18 zur therapeutischen Verwendung, zum Beispiel bei der Behandlung einer Sensitivität auf Ambrosia-Pollen in einem Individuum.

20. Das Protein oder Peptid nach einem der Ansprüche 1 und 4 bis 9, das Allergen nach Anspruch 12 oder 14 oder die Zusammensetzung nach einem der Ansprüche 2, 3 und 18 zur Verwendung in einer *in vivo* Diagnose, zum Beispiel bei Detektion einer Sensitivität auf Ambrosia-Pollen in einem Individuum.

21. Ein monoklonaler oder polyklonaler Antikörper, der gegen ein(e) divergente(s) Region/Epitop eines Amb a I Proteins oder Peptids gerichtet ist und an die in einem der vorausgehenden Ansprüche definierten *Amb a* IA, *Amb a* IB, *Amb a* IC oder *Amb a* ID Proteine oder Peptide spezifisch bindet.

22. Ein monoklonaler oder polyklonaler Antikörper, der gegen ein(e) gewöhnliche(s) Region/Epitop eines *Amb a* I Proteins oder Peptids gerichtet ist und an die in einem der vorausgehenden Ansprüche definierten *Amb a* IA, *Amb a* IB, *Amb a* IC oder *Amb a* ID Proteine oder Peptide spezifisch bindet.

23. Eine Methode zur Detektion einer Sensitivität auf ein Ambrosia-Pollenprotein/peptid in einem Individuum, die Vereinen einer Blutprobe des Individuums mit dem Protein oder Peptid nach einem der Ansprüche 1 und 4 bis 9, dem Allergen nach Anspruch 12 oder 14 oder der Zusammensetzung nach einem der Ansprüche 2, 3 und 18 unter für ein Binden der Blutkomponenten mit dem Protein oder dem Peptid geeigneten Bedingungen und eine Bestimmung des Ausmaßes, mit dem ein Binden erfolgt, umfaßt.

24. Die Methode nach Anspruch 23, worin das Ausmaß, mit dem ein Binden erfolgt, durch eine Bewertung von T-Zellfunktion, T-Zellproliferation, B-Zellfunktion, Binden des Proteins oder des Peptids an im Blut vorhandene Antikörper oder einer Kombination davon bestimmt wird.

## Revendications (Revendications pour l'(les) Etat(s) contractant(s) suivant(s): AT, BE, CH, LI, DE, DK, FR, GB, IT, LU, NL, SE)

1. Protéine ou peptide isolé capable d'influencer les réponses des lymphocytes B et/ou des lymphocites T à l'ambrosie chez les individus sensibles à l'ambrosie, la protéine ou le peptide étant choisi parmi :
(a) *Amb a* IA exempt d'autres membres de la famille *Amb a* I et comprenant au moins une partie de la séquence d'acides aminés représentée sur la Figure 11 ; ou
(b) *Amb a* IB exempt d'autres membres de la famille *Amb a* I et comprenant au moins une partie de la séquence d'acides aminés représentée sur la Figure 12 ; ou
(c) *Amb a* IC exempt d'autres membres de la famille *Amb a* I et comprenant au moins une partie de la séquence d'acides aminés représentée sur la Figure 13; ou
(d) *Amb a* ID exempt d'autres membres de la famille *Amb a* I et comprenant au moins une partie de la séquence d'acides aminés représentée sur la Figure 14,
pour son utilisation en thérapie, prophylaxie ou de diagnostic.

2. Composition capable d'influencer les réponses des lymphocytes B et/ou des lymphocytes T chez les individus sensibles à l'ambrosie, comprenant une ou des quantités définies d'un ou plusieurs peptides ou protéines *Amb a* I isolés, pour son utilisation en thérapie, prophylaxie ou de diagnostic.

3. Composition de la revendication 2, qui comprend une association de différents peptides ou protéines *Amb a* I.

4. Protéine ou peptide *Amb a* I recombinant isolé pour son utilisation en thérapie, prophylaxie ou établissement de diagnostic.

5. Protéine, peptide ou composition de l'une quelconque des revendications précédentes, où :
(a) la protéine ou le peptide est *Amb a* IA tel que représente sur la Figure 11 ; ou
(b) la protéine ou le peptide est *Amb a* IB tel que représente sur la Figure 12 ; ou
(c) la protéine ou le peptide est *Amb a* IC tel que représenté sur la Figure 13 ; ou
(d) la protéine ou le peptide est *Amb a* ID tel que représenté sur la Figure 14 ; ou
(e) la protéine ou le peptide est un variant polymorphique de l'un quelconque de (a) à (d).

6. Protéine, peptide ou composition de l'une quelconque des revendications précédentes, où la protéine ou le peptide comprend une séquence d'acides aminés choisie parmi :

a) EFPILGGITEVKDNDNSVDFC ;
b) YTVTSDKDDDVANC ;
c) GKADWAENRC ;
d) LENGAIFVASGVDPVLTPEQ ; et
e) GFFQVVNNNYDRWGTYA.

7. Protéine, peptide ou composition de l'une quelconque des revendications 1 à 4, où la protéine ou le peptide *Amb a* I est un variant *Amb a* I de *Ambrosia* t*rifida* (*ambrosie géante*), *Ambrosia dumosa* (*ambrosie* du *désert*) ou *Ambrosia psilostachya* (*ambrosie occidentale*).

8. Protéine, peptide ou composition de l'une quelconque des revendications précédentes, où la protéine ou le peptide est capable d'influencer les réponses des lymphocytes B et/ou des lymphocytes T chez les individus sensibles à l'ambrosie et comprend ou consiste essentiellement en au moins un épitope reconnu par des lymphocytes B et/ou des lymphocytes T.

9. Protéine, peptide ou composition de l'une quelconque des revendications précédentes, où la protéine ou le peptide a été modifié pour altérer l'immunogénicité et/ou réduire l'allergénicité.

10. Procédé de production d'un allergène de nature protéique ou peptidique d'ambrosie comprenant les étapes suivantes :
(a) identifier une séquence d'ADN codant pour l'allergène d'ambrosie qui s'hybride par exemple dans des conditions de forte stringence) avec la séquence d'ADN représentée sur la Figure 11, 12, 13 ou 14;
(b) insérer l'ADN identifié à l'étape (a) dans un vecteur d'expression ;
(c) introduire le vecteur d'expression de l'étape (b) dans une cellule hôte ;
(d) cultiver la cellule hôte de l'étape (c) dans des conditions convenant à l'expression de l'ADN inséré dans l'étape (b) ; et
(e) recueillir le produit exprimé.

11. Procédé de la revendication 10, dans lequel la séquence d'ADN identifiée à l'étape (a) présente plus de 40 % d'homologie avec les séquences d'ADN représentées sur la Figure 11, 12, 13 ou 14.

12. Allergène d'ambrosie par exemple *Ambrosia elatior (ambrosie courte), Ambrosia psilostachya (ambrosie occidentale) ou Ambrosia trifida (ambrosie géante) )* produit par le procédé de la revendication 11.

13. Procédé pour préparer un dérivé ou anologue d'un allergène d'ambrosie comprenant les étapes suivantes :
(a) obtenir la protéine ou le peptide tel que défini dans l'une quelconque des revendications 1 à 9 ou l'allergène de la revendication 12 ;
(b) modifier la protéine, le peptide ou l'allergène de l'étape (a) pour produire un dérivé ou analogue de celui-ci.

14. Dérivé ou analogue d'allergène d'ambrosie, produit par le procédé de la revendication 13.

15. Acide nucléique isolé comprenant une séquence nucléotidique qui code pour une protéine ou un peptide *Amb a* IA, *Amb a* IB, *Amb a* IC ou *Amb a* ID.

16. Acide nucléique de la revendication 15, comprenant la région codante représentée sur la Figure 11, 12, 13 ou 14.

17. Acide nucléique de la revendication 16, comprenant la séquence nucléotidique représentée sur la Figure 11, 12, 13 ou 14.

18. Composition comprenant la protéine ou le peptide de l'une quelconque des revendications 1 et 4 à 9 ou l'allergène de la revendication 12 ou 14 et un tampon, un support et/ou un adjuvant.

19. Protéine ou peptide de l'une quelconque des revendications 1 et 4 à 9, allergène de la revendication 12 ou 14 ou composition de l'une quelconque des revendications 2, 3 et 18, pour son utilisation en thérapie, par exemple dans le traitement de la sensibilité d'un individu au pollen d'ambrosie.

20. Protéine ou peptide de l'une quelconque des revendications 1 et 4 à 9, allergène de la revendication 12 ou 14 ou composition de l'une quelconque des revendications 2, 3 et 18, pour son utilisation en diagnostic *in vivo*, par exemple pour détecter la sensibilité d'un individu au pollen. d'ambrosie.

21. Anticorps monoclonal ou polyclonal dirigé contre une région divergente/un épitope divergent d'une protéine ou d'un peptide *Amb a* I, qui se lie spécifiquement aux protéines ou peptides *Amb a* IA, *Amb a* IB, *Amb a* IC ou *Amb a ID* définies dans l'une quelconque des revendications précédentes.

22. Anticorps monoclonal ou polyclonal dirigé contre une région commune/un épitope commun d'une protéine ou d'un peptide *Amb a* I, qui se lie spécifiquement aux protéines ou peptides *Amb a* IA, *Amb a* IB, *Amb a* IC et *Amb a* ID définis dans l'une quelconque des revendications précédentes.

23. Méthode pour détecter la sensibilité d'un individu à une protéine/un peptide de pollen d'ambrosie, comprenant la mise en contact d'un échantillon de sang de l'individu avec la protéine ou le peptide de l'une quelconque des revendications 1 et 4 à 9, l'allergène de la revendication 12 ou 14 ou la composition de l'une quelconque des revendications 2, 3 et 18, dans des conditions appropriées pour la liaison de composants du sang avec la protéine ou le peptide, et la détermination du degré auquel se produit la liaison.

24. Méthode de la revendication 23, dans laquelle le degré auquel se produit la liaison est déterminé par l'évaluation de la fonction les lymphocytes T, de la prolifération des lymphocytes T, de la fonction des lymphocytes B, de la liaison de la protéine ou du peptide aux anticorps présents dans le sang, ou d'une combinaison de celles-ci.

## Revendications (Revendications pour l'(les) Etat(s) contractant(s) suivant(s): ES)

1. Isolement d'une protéine ou d'un peptide capable d'influencer les réponses des lymphocytes B et/ou des lymphocytes T à l'ambroise chez les individus sensibles à l'ambrosie, la protéine ou le peptide étant choisi parmi :
(a) *Amb a* IA exempt d'autres membres de la famille *Amb a* I et comprenant au moins une partie de la séquence d'acides aminés représentée sur la Figure 11 ; ou
(b) *Amb a* IB exempt d'autres membres de la famille A*mb a* I et comprenant au moins une partie de la séquence d'acides aminés représentée sur la Figure 12 ; ou
(c) *Amb a* IC exempt d'autres membres de la famille *Amb a* I et comprenant au moins une partie de la séquence d'acides aminés représentée sur la Figure 13 ;ou
(d) *Amb a* ID exempt d'autres membres de la famille *Amb a* I et comprenant au moins une partie de la séquence d'acides aminés représentée sur la Figure 14,
pour son utilisation en thérapie, prophylaxie ou diagnostic.

2. Procédé de préparation d'une composition capable d'influencer les réponses des lymphocytes B et/ou des lymphocytes T chez les individus sensibles à l'ambrosie, comprenant le mélange de quantité(s) définie(s) d'un ou plusieurs peptides ou protéines *Amb a* I isolés, avec un tampon, support et/ou adjuvant approprié, pour l'utiliser en thérapie, prophylaxie ou diagnostic.

3. Procédé de la revendication 2, qui comprend une association de différents peptides ou protéines *Amb a* I.

4. Isolement d'une protéine ou d'un peptide *Amb a* I recombinant pour son utilisation en thérapie, prophylaxie ou établissement de diagnostic.

5. Isolement ou procédé de l'une quelconque des revendications précédentes, dans lequel :
(a) la protéine ou le peptide est *Amb a* IA tel que représenté sur la Figure 11 ; ou
(b) la protéine ou le peptide est *Amb a* IB tel que représenté sur la Figure 12 ; ou
(c) la protéine ou le peptide est *Amb a* IC tel que représenté sur la Figure 13; ou
(d) la protéine ou le peptide est *Amb a* ID tel que représente sur la Figure 14; ou
(e) la protéine ou le peptide est un variant polymorphique de l'un quelconque de (a) à (d).

6. Isolement du procédé de l'une quelconque des revendications précédentes, dans lequel la protéine ou le peptide comprend une séquence d'acides aminés choisie parmi :

a) EFPILGGITEVKDNDNSVDFC ;
b) YTVTSDKDDDVANC ;
c) GKADWAENRC ;
d) LENGAIFVASGVDPVLTPEQ ; et
e) GFFQVVNNNYDRWGTYA.

7. Isolement ou procédé de l'une quelconque des revendications 1 à 4, dans lequel la protéine ou le peptide *Amb a* I est un variant *Amb a* I de *Ambrosia trifida (ambrosie géante), Ambrosia dumosa (ambrosie du désert) ou Ambrosia psilostachya (ambrosie occidentale)*.

8. Isolement ou procédé de l'une quelconque des revendications précédentes, dans lequel la protéine ou le peptide est capable d'influencer les réponses des lymphocytes B et/ou des lymphocytes T chez les individus sensibles à l'ambrosie et comprend ou consiste essentiellement en au moins un épitope reconnu par des lymphocytes B et/ou des lymphocytes T.

9. Isolement ou procédé de l'une quelconque des revendications précédentes, dans lequel la protéine ou le peptide a été modifié pour altérer l'immunogénicité et/ou réduire l'allergénicité.

10. Procédé de production d'un allergène protéique/peptidique d'ambrosie comprenant les étapes suivantes :
(a) identifier une séquence d'ADN codant pour l'allergène d'ambrosie qui s'hybride par exemple dans des conditions de forte stringence) avec la séquence d'ADN représentée sur la Figure 11, 12, 13 ou 14 ;
(b) insérer l'ADN identifié à l'étape (a) dans un vecteur d'expression ;
(c) introduire le vecteur d'expression de l'étape (b) dans une cellule hôte ;
(d) cultiver la cellule hôte de l'étape (c) dans des conditions convenant à l'expression de l'ADN inséré dans l'étape (b) ; et
(e) recueillir le produit exprimé.

11. Procédé de la revendication 10, dans lequel la séquence d'ADN identifiée à l'étape (a) présente plus de 40 % d'homologie avec les séquences d'ADN représentées sur la Figure 11, 12, 13 ou 14.

12. Procédé pour préparer un dérivé ou anologue d'un allergène d'ambrosie comprenant les étapes suivantes :
(a) obtenir la protéine ou le peptide tel que défini dans l'une quelconque des revendications 1 à 9 ou l'allergène de la revendication 12 ;
(b) modifier la protéine, le peptide ou l'allergène de l'étape (a) pour produire un dérivé ou anologue de celui-ci.

13. Isolement d'un acide nucléique comprenant une séquence nucléotidique qui code pour une protéine ou un peptide *Amb a* IA, *Amb a* IB, *Amb a* IC ou *Amb a* ID.

14. Isolement de la revendication 13, dans lequel l'acide nucléique comprend la région codante représentée sur la Figure 11, 12, 13 ou 14.

15. Isolement de la revendication 14, dans lequel l'acide nucléique comprend la séquence nucléotidique représentée sur la Figure 11, 12, 13 ou 14.

16. Procédé de préparation d'une composition, comprenant le mélange de la protéine ou du peptide de l'une quelconque des revendications 1 et 4 à 9 ou d'un allergène préparé par le procédé de la revendication 10 ou 12 avec un tampon, un support et/ou un adjuvant.

17. Utilisation de la protéine ou du peptide de l'une quelconque des revendications 1 et 4 à 9, de l'allergène préparé par le procédé de la revendication 10 ou 12 ou de la composition préparée par le procédé de l'une quelconque des revendications 2, 3 et 16, pour la fabrication d'un médicament à utiliser en thérapie, par exemple dans le traitement de la sensibilité d'un individu au pollen d'ambrosie.

18. Utilisation de la protéine ou du peptide de l'une quelconque des revendication 1 et 4 à 9, de l'allergène préparé par le procédé de la revendication 10 ou 12 ou de la composition préparée par le procédé de l'une quelconque des revendications 2, 3 et 16, pour la fabrication d'un médicament à utiliser dans diagnostic *in vivo*, par exemple pour détecter la sensibilité d'un individu au pollen d'ambrosie.

19. Fabrication d'un anticorps monoclonal ou polyclonal dirigé contre une région divergente/un épitope divergent d'une protéine ou d'un peptide *Amb a* I, qui se lie spécifiquement aux protéines ou peptides *Amb a* IA, *Amb a* IB, *Amb a* IC ou *Amb a* ID définis dans l'une quelconque des revendications précédentes.

20. Fabrication d'un anticorps monoclonal ou polyclonal dirigé contre une région commune/un épitope commun d'une protéine ou d'un peptide *Amb a* I, qui se lie spécifiquement aux protéines ou peptides *Amb a* IA, *Amb a* IB, *Amb a* IC et *Amb a* ID définis dans l'une quelconque des revendications précédentes.

21. Méthode pour détecter la sensibilité d'un individu à une protéine/un peptide de pollen d'ambrosie, comprenant la mise en contact d'un échantillon de sang de l'individu avec la protéine ou le peptide de l'une quelconque des revendications 1 et 4 à 9, l'allergène préparé par le procédé de la revendication 10 ou 12 ou la composition préparée par le procédé de l'une quelconque des revendications 2, 3 et 16, dans des conditions appropriées cour la liaison de composants du sang avec la protéine ou le peptide, et la détermination du degré auquel se produit la liaison.

22. Méthode de la revendication 21, dans laquelle le degré auquel se produit la liaison est déterminé par l'évaluation de la fonction des lymphocytes T, de la prolifération des lymphocytes T, de la fonction des lymphocytes B, de la liaison de la protéine ou du peptide aux anticorps présents dans le sang, ou d'une combinaison de celles-ci.
